# EUROPÄISCHE PATENTANMELDUNG

(11) **EP 2 589 293 A1**
(43) Veröffentlichungstag der Anmeldung: **08.05.2013**
(21) Anmeldenummer: 11187674.4
(22) Anmeldetag: 03.11.2011
(51) Int. Cl.: A01N 43/653, A01N 43/713, C07D 249/14, C07D 257/06, C07D 401/12

(54) **Herbizid-Safener-Zusammensetzungen enthaltend N-(Tetrazol-5-yl)- und N-(Triazol-5-yl)arylcarbonsäureamide**

(71) Anmelder: Bayer CropScience AG, 40789 Monheim (DE)
(72) Erfinder: Die Erfindernennung liegt noch nicht vor

(57) **Zusammenfassung**

Es werden Herbizid-Safener-Zusammensetzungen beschrieben, die herbizide Wirkstoffe aus der Gruppe der N-(Tetrazol-5-yl)- und N-(Triazol-5-yl)arylcarbonsäureamide sowie Safener enthalten. Diese Herbizid-Safener-Zusammensetzungen sind für den Einsatz gegen Schadpflanzen in Nutzpflanzenkulturen besonders geeignet.

## Beschreibung

Die vorliegende Erfindung betrifft agrochemisch wirksame Herbizid-Safener-Zusammensetzungen, Verfahren zu deren Herstellung sowie deren Verwendung zur Bekämpfung von Schadpflanzen.

Aus den prioritätsälteren und nicht vorveröffentlichten EP 101748937 und EP111763785 ist bekannt, dass bestimmte N-(Tetrazol-5-yl)- und N-(Triazol-5-yl)arylcarbonsäureamide herbizide Eigenschaften besitzen und ein breites Spektrum von Unkräutern bekämpfen. Jedoch sind diese Wirkstoffe zum Teil nicht voll verträglich mit wichtigen Kulturpflanzen, wie Getreidearten, Mais oder Reis. Sie können deshalb in manchen Kulturen nicht so eingesetzt werden, dass die erwünschte breite herbizide Wirksamkeit gegenüber Schadpflanzen gewährleistet ist.

Aufgabe der vorliegenden Erfindung ist es daher, herbizide Mittel zu bereitzustellen, in welchen die Selektivität der oben genannten Herbizide gegenüber wichtigen Kulturpflanzen erhöht ist. Diese Aufgabe wird durch die nachfolgend beschriebenen erfindungsgemäßen Zusammensetzungen enthaltend Herbizide und Safener gelöst.

Gegenstand der vorliegenden Erfindung sind Zusammensetzungen, enthaltend
(A) eine oder mehrere Verbindungen der Formel (I) oder deren Salze, 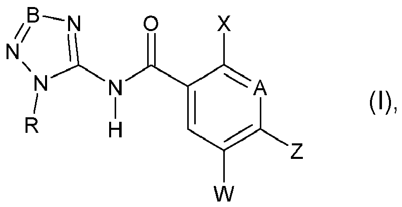 worin die Symbole und Indizes folgende Bedeutungen haben:
   A bedeutet N oder CY,
   B bedeutet N oder CH,
   X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
   Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-AlkylS(O)ₙR², (C₁-C₆)-Alkyl-OR¹, (C₁-C₆)-Alkyl-OCOR¹, (C₁-C₆)-Alkyl-OSO2R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹O₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
   Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R, N(R¹)₂, P(O)(OR⁵)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt, oder
   Z kann auch Wasserstoff, (C₁-C₆)-Alkyl oder (C₁-C₆)-Alkoxy bedeuten, falls Y für den Rest S(O)ₙR² steht,
   W bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₇)-Cycloalkyl, (C₃-C₇)-Halogencycloalkyl, (C₁-C₆)-Alkoxy, (C₁-C₆)-Halogenalkoxy, S(O)ₙ-(C₁-C₆)-Alkyl, S(O)ₙ-(C₁-C₆)-Halogenalkyl, (C₁-C₆)-Alkoxy-(C₁-C₄)-alkyl, (C₁-C₆)-Alkoxy-(C₁-C₄)-halogenalkyl, Halogen, Nitro, NR³COR³ oder Cyano,
   R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Hydroxy, Nitro, Cyano, SiR⁵₃, PO(OR⁵)₂, S(O)ₙ-(C₁-C₆)-Alkyl, S(O)ₙ-(C₁-C₆)-Halogenalkyl, (C₁-C₆)-Alkoxy, Halogen-(C₁-C₆)-alkoxy, N(R³)₂, COR³, COOR³, OCOR³, NR³COR³, NR³SO₂R⁴, O(C₁-C₂)-Alkyl-(C₃-C₆)-Cycloalkyl, (C₃-C₆)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder R bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl, wobei Heterocyclyl n Oxogruppen trägt,
   Q bedeutet O, S, oder NR³,
   R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl wobei die 21 letztgenannten Reste jeweils durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
   R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 21 letztgenannten Reste jeweils durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
   R³ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
   R⁴ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
   R⁵ bedeutet (C₁-C₄)-Alkyl, n bedeutet 0, 1 oder 2,
   s bedeutet 0, 1, 2 oder 3,
   sowie
(B) einen oder mehrere Safener.

Die Bezeichnungen "Herbizde (A)" und "Verbindungen der allgemeinen Formel (I)" sind in vorliegender Anmeldung als gleichbedeutend zu verstehen.

Die erfindungsgemäßen Herbizid-Safener-Zusammensetzungen können zusätzliche weitere Komponenten, beispielsweise Pflanzenschutzmittelwirkstoffe anderer Art und/oder im Pflanzenschutz übliche Zusatzstoffe und/oder Formulierungshilfsmittel, enthalten oder zusammen mit diesen eingesetzt werden.

Die Herbizide (A) und die Safener (B) können auf bekannte Weise angewendet werden, beispielsweise gemeinsam (beispielsweise als Co-Formulierung oder als Tank-Mischung) oder auch zeitlich versetzt (Splitting), z.B. auf die Pflanzen, Pflanzenteile, Pflanzensamen oder die Fläche, auf der die Pflanzen wachsen. Möglich ist z.B. die Anwendung der Einzelwirkstoffe oder der Herbizid-Safener-Zusammensetzung in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Zusammensetzung. Möglich ist auch die Anwendung der Einzelwirkstoffe oder der Herbizid-Safener-Zusammensetzung zur Saatgutbehandlung.

Bevorzugt sind solche erfindungsgemäße Zusammensetzungen, die als Herbizid (A) solche Verbindungen der allgemeinen Formel (I) und deren Salze enthalten, worin
A bedeutet N oder CY,
B bedeutet N oder CH,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR¹, OCOR¹, OSO₂R², S(O)ₙR² SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, OR¹, COOR¹, OSO₂R², S(O)ₙR², SO₂OR¹, SO₂ N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder Z kann auch Wasserstoff, (C₁-C₆)-Alkyl oder (C₁-C₆)-Alkoxy bedeuten, falls Y für den Rest S(O)ₙR² steht,
W bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Nitro, Cyano, SiR⁵₃, P(OR⁵)₃, S(O)ₙ-(C₁-C₆)-Alkyl, (C₁-C₆)-Alkoxy, Halogen-(C₁-C₆)-alkoxy, N(R³)₂, COR³, CO0R³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder
R bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl, Q bedeutet O, S, oder NR³,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die sechzehn letztgenannten Reste jeweils durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese sechzehn letztgenannten Reste jeweils durch s Reste aus der Gruppe bestehend aus Cyano, Halogen, Nitro, OR³, S(O)ₙR⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl , (C₂-C₆)-Alkenyl oder (C₂-C₆)-Alkinyl,
R⁵ bedeutet Methyl oder Ethyl,
n bedeutet 0, 1 oder 2, und
s bedeutet 0, 1, 2 oder 3.

Bevorzugt sind auch erfindungsgemäße Zusammensetzungen, die als Herbizid (A) solche Verbindungen der allgemeinen Formel (I) und deren Salze enthalten, worin
A bedeutet N oder CY,
B bedeutet N oder CH,
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, (cd-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Halogen-(C₁-C₆)-alkyl, (C₃-C₆)-Cycloalkyl, S(O)ₙR², 1,2,4-Triazol-1-yl, oder Z kann auch Wasserstoff, Methyl, Methoxy oder Ethoxy bedeuten, falls Y für den Rest S(O)ₙR² steht,
W bedeutet Wasserstoff, Methyl, Ethyl, Methoxymethyl, Methoxy, Fluor, Chlor oder S(O)ₙCH₃,
R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Cyano, S(O)ₙ-(C₁-C₆)-Alkyl, (C₁-C₆)-Alkoxy, Halogen-(C₁-C₆)-alkoxy, COR³, COOR³, OCOR³, NR³COR³, NR³SO₂R⁴, (C₃-C₆)-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt, oder R bedeutet durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Phenyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die sechzehn letztgenannten Reste jeweils durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet jeweils durch s Reste aus der Gruppe bestehend aus Halogen und OR³ substituiertes (C₁-C₆)-Alkyl, (C₃-C₆)-Cycloalkyl oder (C₃-C₆)-Cycloalkyl-(C₁-C₆)-alkyl,
R³ bedeutet Wasserstoff oder (C₁-C₆)-Alkyl,
R⁴ bedeutet (C₁-C₆)-Alkyl,
R⁵ bedeutet Methyl oder Ethyl,
n bedeutet 0, 1 oder 2, und
s bedeutet 0, 1, 2 oder 3.

Besonders bevorzugt sind solche erfindungsgemäße Zusammensetzungen, die als Herbizid (A) Verbindungen der allgemeinen Formel (I) und deren Salze enthalten, worin
A bedeutet CY,
B bedeutet N,
X bedeutet Chlor, Methyl, Ethyl, Propyl, cyclo-Propyl, Methoxy oder SO₂CH₃,
Y bedeutet Wasserstoff, CH₂OCH₃, CH₂OCH₂CF₃, CH₂OC₂H₄OCH₃, 4,5-Dihydro-1,2-oxazol-3-yl, 5-Methoxymethy-4,5-dihydro-1,2-oxazol-3-yl, Pyrazol-1-yl, OMe, OEt, OPr, OiBu, OCH₂cPr, OC₂H₄OCH₃, SO₂ CH₃, S(O)CH₃ oder SCH₃, SO₂Et, S(O)Et oder SEt,
Z bedeutet Trifluormethyl, SO₂CH₃, SO₂Et, Chlor oder Brom,
W bedeutet Wasserstoff, und
R bedeutet Methyl, Ethyl, Propyl oder Methoxyethyl.

Ganz besonders bevorzugt sind erfindungsgemäße Zusammen-setzungen, die als Herbizid (A) solche Verbindungen der allgemeinen Formel (I) und deren Salze enthalten, worin
- A: bedeutet CY,
- B: bedeutet N
- X: bedeutet Chlor,
- Y: SO₂CH₃, SOCH₃ oder SO₂Et,
- Z: bedeutet Wasserstoff oder Methyl,
- W: bedeutet Wasserstoff, und
- R: bedeutet Methyl.

Ganz besonders bevorzugt sind auch erfindungsgemäße Zusammen-setzungen, die als Herbizid (A) solche Verbindungen der allgemeinen Formel (I) und deren Salze enthalten, worin
- A: bedeutet CY,
- B: bedeutet N
- X: bedeutet Chlor oder Brom,
- Y: bedeutet Wasserstoff, Methyl, SO₂CH₃ oder SCH₃,
- Z: bedeutet Wasserstoff, SO₂CH₃ oder SCH₃,
- W: bedeutet Methyl, und
- R: bedeutet Methyl oder Ethyl.

Ganz besonders bevorzugt sind auch solche erfindungsgemäße Zusammensetzungen, die als Herbizid (A) Verbindungen der allgemeinen Formel (I) und deren Salze enthalten, worin
- A: bedeutet CY,
- B: bedeutet CH,
- X: bedeutet Chlor oder Methyl,
- Y: bedeutet 4,5-Dihydro-1,2-oxazol-3-yl, Pyrazol-1-yl, OC₂H₄OCH₃ oder SO₂CH₃,
- Z: bedeutet Trifluormethyl, SO₂CH₃, SO₂Et oder Chlor,
- W: bedeutet Wasserstoff, und
- R: bedeutet Methyl.

Ganz besonders bevorzugt sind auch erfindungsgemäße Zusammen-setzungen, die als Herbizid (A) solche Verbindungen der allgemeinen Formel (I) und deren Salze enthalten, worin
- A: bedeutet N,
- B: bedeutet N,
- X: bedeutet Chlor, Brom, SO₂CH₃, Methoxymethyl, OCH₂cPr, OC₂H₄OCH₃ oder Methyl,
- Z: bedeutet Trifluormethyl,
- W: bedeutet Wasserstoff, und
- R: bedeutet Methyl oder Ethyl.

In der Formel (I) und allen nachfolgenden Formeln können Alkylreste mit mehr als zwei Kohlenstoffatomen geradkettig oder verzweigt sein. Alkylreste bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z. B. Propargyl, But-2-in-1-yl, But-3-in- 1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl,

Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Heteroaryl steht beispielsweise für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, dass diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die in den erfindungsgemäßen Herbizid-Safener-Zusammensetzungen enthaltenen Verbindungen der Formel (I) und die weiter unten beschriebenen Safener können jeweils Salze bilden. Salzbildung kann durch Einwirkung einer Base auf solche Verbindungen der Formel (I) oder Safener erfolgen, die ein acides Wasserstoffatom tragen, z.B. im Falle dass R¹ eine COOH-Gruppe oder eine Sulfonamid-Gruppe NHSO₂ enthält. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere NaOH, KOH, Na₂CO₃, K₂CO₃, NaHCO₃, und KHCO₃. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erd-alkalimetallsalze, insbesondere Natrium- und Kaliumsalze, oder auch Ammonium-salze, Salze mit organischen Aminen oder quartäre (quaternäre) Ammoniumsalze, zum Beispiel mit Kationen der Formel [NRR'R"R'"]⁺, worin R bis R'" jeweils voneinander einen organischen Rest, insbesondere Alkyl, Aryl, Aralkyl oder Alkylaryl darstellen. Geeignet sind auch Alkylsulfonium- und Alkylsulfoxoniumsalze, wie (C₁-C₄)-Trialkylsulfonium- und (C₁-C₄)-Trialkylsulfoxoniumsalze.

Die in den erfindungsgemäßen Herbizid-Safener-Zusammensetzungen enthaltenen Verbindungen der Formel (I) und die weiter unten beschriebenen Safener können durch Anlagerung einer geeigneten anorganischen oder organischen Säure, z.B. Mineralsäuren, wie HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, jeweils Salze bilden. Diese Salze enthalten dann die konjugierte Base der Säure als Anion. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Bei Resten mit Kohlenstoffatomen sind grundsätzlich solche mit 1 bis 6 Kohlenstoffatomen, vorzugsweise 1 bis 4 Kohlenstoffatomen, insbesondere 1 oder 2 Kohlenstoffatomen, bevorzugt.

Beispiele für als Herbizid (A) verwendete Verbindungen sind in den folgenden Tabellen aufgeführt:

Darin bedeuten die verwendeten Abkürzungen: Et = Ethyl Me = Methyl n-Pr = n-Propyl i-Pr = Isopropyl c-Pr = Cyclopropyl Ph = Phenyl Ac = Acetyl i-Bu = Isobutyl

**Tabelle 1: Verbindungen der allgemeinen Formel (I), worin A für CY und B für N und W für Wasserstoff steht**

| | | | | |
|---|---|---|---|---|
| 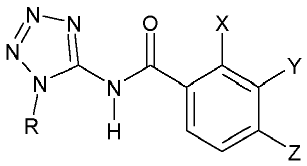 | | | | |

| Bsp.-Nr. | R | X | Y | Z |
|---|---|---|---|---|
| A1-1 | Me | Cl | H | SO₂Me |
| A1-2 | Me | SO₂M | H | CF₃ |
| A1-3 | Me | Me | SMe | CF₃ |
| A1-4 | MeOC₂H₄ | Me | SMe | CF₃ |
| A1-5 | Me | Me | SOMe | CF₃ |
| A1-6 | Et | Me | SOMe | CF₃ |
| A1-7 | Me | Me | SO₂M | CF₃ |
| A1-8 | Et | Me | SO₂M | CF₃ |
| A1-9 | Pr | Me | SO₂Me | CF₃ |
| A1-10 | MeOC₂H₄ | Me | SO₂M | CF₃ |
| A1-11 | Me | Me | SEt | CF₃ |
| A1-12 | Et | Me | SEt | CF₃ |
| A1-13 | Me | Me | SOEt | CF₃ |
| A1-14 | Et | Me | SOEt | CF₃ |
| A1-15 | Me | Me | SO₂E | CF₃ |
| A1-16 | Et | Me | SO₂E | CF₃ |
| A1-17 | Me | Me | SO₂M | Cl |
| A1-18 | Me | Me | SEt | Cl |
| A1-19 | Me | Me | SOEt | Cl |
| A1-20 | Et | Me | SOEt | Cl |
| A1-21 | Me | Me | SO₂Et | Cl |
| A1-22 | Me | Me | SMe | Br |
| A1-23 | Me | Me | SEt | Br |
| A1-24 | Me | Me | 4,5-Dihydro-1,2-oxazol-3-yl | SO₂Me |
| A1-25 | Et | Me | 4,5-Dihydro-1,2-oxazol-3-yl | SO₂Me |
| A1-26 | Me | Me | Pyrazol-1-yl | SO₂Me |
| A1-27 | Et | Me | Pyrazol-1-yl | SO₂Me |
| A1-28 | Me | Me | SMe | SO₂Me |
| A1-29 | Me | Me | SO₂M | SO₂Me |
| A1-30 | Et | Me | SO₂M | SO₂Me |
| A1-31 | Me | Me | SO₂E | SO₂Me |
| A1-32 | Et | Me | SO₂E | SO₂Me |
| A1-33 | Me | Et | SMe | CF₃ |
| A1-34 | Me | Et | SOMe | CF₃ |
| A1-35 | Me | Et | SO₂M | CF₃ |
| A1-36 | Me | Et | SMe | Cl |
| A1-37 | Et | Et | SMe | Cl |
| A1-38 | Me | Et | SOMe | Cl |
| A1-39 | Me | Et | SMe | Br |
| A1-40 | Me | Et | SO₂M | Br |
| A1-41 | Me | Pr | SMe | CF₃ |
| A1-42 | Me | Pr | SOMe | CF₃ |
| A1-43 | Me | c-Pr | SMe | CF₃ |
| A1-44 | Me | OMe | SMe | CF₃ |
| A1-45 | Me | OMe | SOMe | CF₃ |
| A1-46 | Me | OMe | SO₂Me | CF₃ |
| A1-47 | Me | OMe | SEt | CF₃ |
| A1-48 | Me | Cl | SMe | H |
| A1-49 | Me | Cl | SO₂Me | Me |
| A1-50 | Me | Cl | SO₂Et | Me |
| A1-51 | Me | Cl | SO₂Me | CF₃ |
| A1-52 | Me | Cl | OC₂H₄OMe | Cl |
| A1-53 | Me | Cl | SMe | Cl |
| A1-54 | Et | Cl | SMe | Cl |
| A1-55 | Me | Cl | SOMe | Cl |
| A1-56 | Et | Cl | SOMe | Cl |
| A1-57 | Me | Cl | SO₂Me | Cl |
| A1-58 | Et | Cl | SO₂Me | Cl |
| A1-59 | Me | Cl | SO₂Et | Cl |
| A1-60 | Me | Cl | CH₂OMe | SO₂Me |
| A1-61 | Me | Cl | CH₂OCH₂CF₃ | SO₂Me |
| A1-62 | Et | Cl | CH₂OCH₂CF₃ | SO₂Me |
| A1-63 | Me | Cl | CH₂OC₂H₄OMe | SO₂Me |
| A1-64 | Me | Cl | 4,5-Dihydro-1,2-oxazol-3-yl | SO₂Me |
| A1-65 | Me | Cl | 4,5-Dihydro-1,2-oxazol-3-yl | SO₂Et |
| A1-66 | Me | Cl | 5-Methoxymethy-4,5-dihydro-1,2-oxazol-3-yl | SO₂Et |
| A1-67 | Me | Cl | OMe | SO₂Me |
| A1-68 | Me | Cl | OMe | SO₂Et |
| A1-69 | Me | Cl | OEt | SO₂Me |
| A1-70 | Me | Cl | OEt | SO₂Et |
| A1-71 | Me | Cl | OPr | SO₂Me |
| A1-72 | Me | Cl | OPr | SO₂Et |
| A1-73 | Me | Cl | Oi-Bu | SO₂Me |
| A1-74 | Me | Cl | OCH₂c-Pr | SO₂Me |
| A1-75 | Me | Cl | OCH₂c-Pr | SO₂Et |
| A1-76 | Me | Cl | OC₂H₄OMe | SO₂Me |
| A1-77 | Me | Cl | SMe | SO₂Me |

**Tabelle 2: Verbindungen der allgemeinen Formel (I), worin A für CY und B für CH und W für Wasserstoff steht**

| | | | | |
|---|---|---|---|---|
| 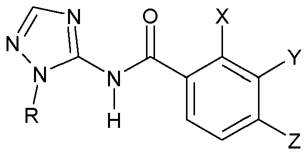 | | | | |

| Bsp.-Nr. | R | X | Y | Z |
|---|---|---|---|---|
| A2-1 | Me | Me | SO₂Me | CF₃ |
| A2-2 | Me | Me | 4,5-Dihydro-1,2-oxazol-3-yl | SO₂Me |
| A2-3 | Me | Me | Pyrazol-1-yl | SO₂Me |
| A2-4 | Me | Me | SO₂Me | SO₂Me |
| A2-5 | Me | Cl | SO₂Me | Cl |
| A2-6 | Me | Cl | 4,5-Dihydro-1,2-oxazol-3-yl | SO₂Me |
| A2-7 | Me | Cl | 4,5-Dihydro-1,2-oxazol-3-yl | SO₂Et |
| A2-8 | Me | Cl | OC₂H₄OMe | SO₂Me |

**Tabelle 3: Verbindungen der allgemeinen Formel (I), worin A für CY und B für N steht**

| | | | | | |
|---|---|---|---|---|---|
| 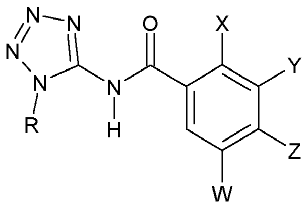 | | | | | |

| Bsp.-Nr. | R | X | Y | Z | W |
|---|---|---|---|---|---|
| A3-1 | Me | Cl | H | SMe | Me |
| A3-2 | Me | Cl | SMe | H | Me |
| A3-3 | Me | Cl | SO₂Me | H | Me |
| A3-4 | Et | Cl | SO₂Me | H | Me |
| A3-5 | Me | Cl | Me | SMe | Me |
| A3-6 | Et | Cl | Me | SO₂Me | Me |
| A3-7 | Me | Br | SO₂Me | H | Me |

**Tabelle 4: Verbindungen der allgemeinen Formel (I), worin A für N und B für N und W für Wasserstoff steht**

| | | | |
|---|---|---|---|
| 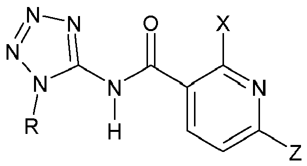 | | | |

| Bsp.-Nr. | R | X | Z |
|---|---|---|---|
| A4-1 | Me | Me | CF₃ |
| A4-2 | Me | CH₂OMe | CF₃ |
| A4-3 | Et | CH₂OMe | CF₃ |
| A4-4 | Me | CH₂OC₂H₄OMe | CF₃ |
| A4-5 | Et | CH₂OC₂H₄OMe | CF₃ |
| A4-6 | Me | CH₂OCH₂c-Pr | CF₃ |
| A4-7 | Me | Cl | CF₃ |
| A4-8 | Me | Br | CF₃ |
| A4-9 | Me | SO₂Me | CF₃ |

Die Aufwandmenge der Herbizide (A) kann mit den äußeren Bedingungen wie Temperatur, Feuchtigkeit, der Art des verwendeten Herbizids in einem weiten Bereich variieren, z.B. zwischen 0,001 g und 2000 g a.i./ha (ai/ha bedeutet dabei im Folgenden Aktivsubstanz pro Hektar" = bezogen auf 100%igen Wirkstoff).

Bei Anwendungen mit Aufwandmengen von 0,01 g bis 1000 g a.i./ha der Herbizide (A) wird im Vor- und Nachauflaufverfahren ein relativ breites Spektrum an Schadpflanzen bekämpft, z.B. annuellen und perennierenden mono- oder dikotylen Unkräutern sowie an unerwünschten Kulturpflanzen. Bei den erfindungsgemäßen Zusammensetzungen liegen die Aufwandmengen in der Regel niedriger, z. B. im Bereich von 0,1 g bis 800 g a.i./ha, vorzugsweise 1 g bis 500 g a.i./ha, besonders bevorzugt 10 g bis 400 g a.i./ha.

Die Herbizide (A) sind zur Bekämpfung von Schadpflanzen, z.B. in Pflanzenkulturen geeignet, beispielsweise in wirtschaftlich bedeutenden Ackerbaukulturen z.B. monokotylen Ackerbaukulturen wie Getreide (z.B. Weizen, Gerste, Roggen, Hafer), Reis, Mais, Hirse, oder dikotylen Ackerbaukulturen wie Zuckerrübe, Raps, Baumwolle, Sonnenblume und Leguminosen z.B. der Gattungen Glycine (z.B. Glycine max. (Soja) wie nicht-transgene Glycine max. (z.B. konventionelle Sorten wie STS-Sorten) oder transgene Glycine max. (z.B. RR-Soja oder LL-Soja) und deren Kreuzungen), Phaseolus, Pisum, Vicia und Arachis, oder Gemüsekulturen aus verschiedenen botanischen Gruppen wie Kartoffel, Lauch, Kohl, Karotte, Tomate, Zwiebel, sowie Dauer- und Plantagenkulturen wie Kern- und Steinobst, Beerenobst, Wein, Hevea, Bananen, Zuckerrohr, Kaffee, Tee, Citrus, Nussplantagen, Rasen, Palmenkulturen und Forstkulturen. Für die Anwendung der erfindungsgemäßen Herbizid-Safener-Zusammensetzungen (A)+(B) sind diese Kulturen ebenfalls bevorzugt, besonders bevorzugt ist der Einsatz in Getreide (z.B. Weizen, Gerste, Roggen, Hafer), Reis, Mais, Hirse, Zuckerrübe, Zuckerrohr, Sonnenblume, Raps und Baumwolle. Die Herbizid-Safener- Zusammensetzungen (A)+(B) sind auch einsetzbar in toleranten und nicht toleranten Mutantenkulturen und toleranten und nicht toleranten transgenen Kulturen, vorzugsweise von Mais, Reis, Getreide, Raps und Soja, z.B. solche die gegen Imidazolinon-Herbizide, Atrazin, Glufosinate oder Glyphosate resistent sind.

Die Herbizide (A) sind aus EP 0 173 657 A1 und der prioritätsälteren nicht veröffentlichten EP 09012169.0 bekannt und können nach den dort beschriebenen Verfahren erhalten werden.

Unter den als Komponente (B) enthaltenen Safenern werden Verbindungen verstanden, die geeignet sind, phytotoxische Wirkungen von Pflanzenschutzmittelwirkstoffen wie Herbiziden an Kulturpflanzen zu reduzieren.

Im Rahmen der vorliegenden Erfindung werden die Herbizide (A) mit folgenden Safener-Verbindungen kombiniert:
S1) Verbindungen aus der Gruppe heterocyclischer Carbonsäurederivate:
   S1^{a}) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91 /07874 beschrieben sind;
   S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333 131 und EP-A-269 806 beschrieben sind;
   S1 ^{c}) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-1H-1,2,4-triazol-3-carbonsäureethylester (S1-7), und verwandte Verbindungen, wie sie in EP-A-174 562 und EP-A-346 620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91 /08202 beschrieben sind, bzw. 5,5-Diphenyl-2-isoxazolin-carbonsäure (S1-10) oder 5,5-Diphenyl-2-isoxazolincarbonsäureethylester (S 1-11) ("Isoxadifen-ethyl") oder -n-propylester (S 1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Verbindungen aus der Gruppe der 8-Chinolinyloxyderivate (S2):
   S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocet-mexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) (synonym: "BAS145138" oder "LAB145138") (RS)-1-Dichloracetyl-3,3,8a-trimethylperhydropyrrolo[1,2-a]pyrimidin-6-on der Firma BASF (S3-9), "Furilazol" oder "MON 13900" ((RS)-3-Dichloracetyl-5-(2-furyl)-2,2-dimethyloxazolidin) (S3-10), sowie dessen (R)-Isomer (S3-11).
S4) Verbindungen aus der Klasse der Acylsulfonamide (S4):
   S4^{a}) N-Acylsulfonamide der Formel (S4^{a}) und deren Salze wie sie in der WO-A-97/45016 beschrieben sind, 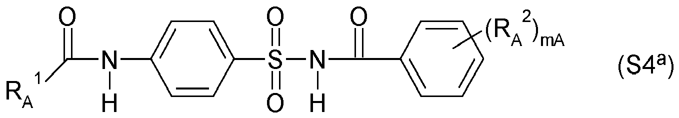 worin
      R_{A}¹ bedeutet (C₁-C₆)-Alkyl oder (C₃-C₆)-Cycloalkyl, wobei diese Reste durch V_{A} Substituenten aus der Gruppe Halogen, (C₁-C₄)-Alkoxy, (C₁-C₆)-Haloalkoxy und (C₁-C₄)-Alkylthio und im Falle cyclischer Reste auch durch (C₁-C₄)-Alkyl und (C₁-C₄)-Haloalkyl substituiert sind;
      R_{A}² bedeutet Halogen, (C₁-C₄)-Alkyl, (C₁-C₄)-Alkoxy, CF₃;
      m_{A} bedeutet 1 oder 2;
      V_{A} bedeutet 0,1,2 oder 3.
   S4^{b}) Verbindungen vom Typ der 4-(Benzoylsulfamoyl)benzamide der Formel (S4^{b}) und deren Salze, wie sie in der WO-A-99/16744 beschrieben sind, 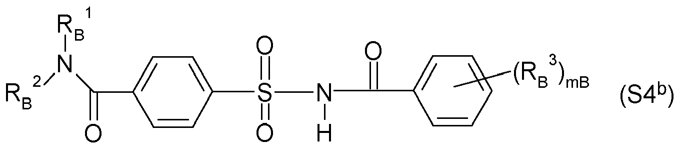 worin
      R_{B}¹, R_{B}² bedeuten unabhängig voneinander Wasserstoff, (C₁-C₆)-Alkyl, (C₃-C₆)-Cycloalkyl, (C₃-C₆)-Alkenyl oder (C₃-C₆)-Alkinyl,
      R_{B}³ bedeutet Halogen, (C₁-C₄)-Alkyl, (C₁-C₄)-Haloalkyl oder (C₁-C₄)-Alkoxy und
      m_{B} bedeutet 1 oder 2,
      beispielsweise solche worin
      R_{B}¹ = Cyclopropyl, R_{B}² = Wasserstoff und (R_{B}³) = 2-OMe ist (S4-1, "Cyprosulfamide"),
      R_{B}¹ = Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B}³) = 2-OMe ist (S4-3),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5).
   S4^{c}) Verbindungen aus der Klasse der Benzoylsulfamoylphenylharnstoffe der Formel (S4^{c}), wie sie in der EP-A-365484 beschrieben sind 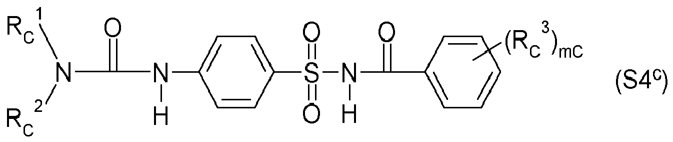 worin
      R_{C}¹, R_{C}² bedeuten unabhängig voneinander Wasserstoff, (C₁-C₈)-Alkyl, (C₃-C₈)-Cycloalkyl, (C₃-C₆)-Alkenyl oder (C₃-C₆)-Alkinyl,
      R_{C}³ bedeutet Halogen, (C₁-C₄)-Alkyl, (C₁-C₄)-Alkoxy oder CF₃;
      m_{C} bedeutet 1 oder 2;
      beispielsweise
      1-[4-(N-2-Methoxybenzoylsulfamoyl)phenyl]-3-methylharnstoff (S4-6), 1-[4-(N-2-Methoxybenzoylsulfamoyl)phenyl]-3,3-dimethylharnstoff, 1-[4-(N-4,5-Dimethylbenzoylsulfamoyl)phenyl]-3-methylharnstoff.
   S4^{d}) Verbindungen vom Typ der N-Phenylsulfonylterephthalamide der nachfolgenden Formel (S4^{d}) unde deren Salze, die z.B. bekannt sind aus CN 101838227, 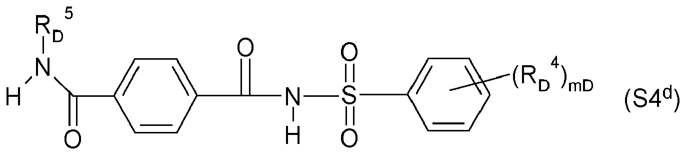 worin
      R_{D}⁴ bedeutet Halogen, (C₁-C₄)-Alkyl, (C₁-C₄)-Alkoxy oder CF₃;
      m_{D} 1 oder 2;
      R_{D}⁵ bedeutet Wasserstoff, (C₁-C₆)-Alkyl, (C₃-C₆)-Cycloalkyl, (C₂-C₆)-Alkenyl, (C₂-C₆)-Alkinyl oder (C₅-C₆)-Cycloalkenyl.
S5) Wirkstoffe aus der Klasse der Hydroxyaromaten und der aromatischaliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydrochinoxalin-2-on, wie sie in der WO-A-2005/112630 beschrieben sind.
S7) Verbindungen aus der Klasse der Diphenylmethoxyessigsäurederivate (S7), z. B. Diphenylmethoxyessigsäuremethylester(CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8),wie sie in der WO-A-98/27049 beschrieben sind 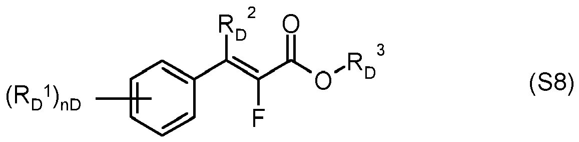 worin
   R_{D}¹ bedeutet Halogen, (C₁-C₄)-Alkyl, (C₁-C₄)-Haloalkyl, (C₁-C₄)-Alkoxy, (C₁-C₄)-Haloalkoxy,
   R_{D}² bedeutet Wasserstoff oder (C₁-C₄)-Alkyl
   R_{D}³ bedeutet Wasserstoff, (C₁-C₈)-Alkyl, (C₂-C₄)-Alkenyl, (C₂-C₄)-Alkinyl oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze
   n_{D} bedeutet 0, 1 oder 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}) wie sie in der WO-A-2007/023719 und WO-A-2007/023764 beschrieben sind 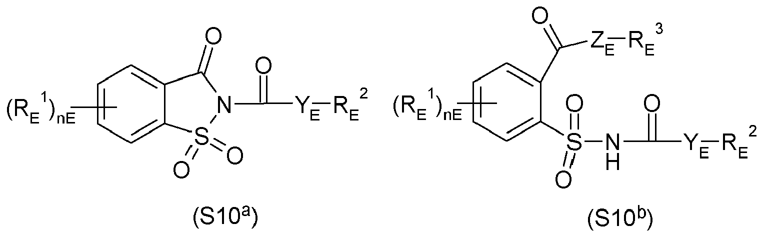 worin
   R_{E} ¹ bedeutet Halogen, (C₁-C₄)-Alkyl, Methoxy, Nitro, Cyano, CF₃, OCF₃
   Y_{E}, Z_{E} bedeuten unabhängig voneinander O oder S,
   n_{E} bedeutet 0, 1, 2, 3 oder 4,
   R_{E}² bedeutet (C₁-C₁₆)-Alkyl, (C₂-C₆)-Alkenyl, (C₃-C₆)-Cycloalkyl, Aryl, Benzyl oder Halogenbenzyl,
   R_{E}³ bedeutet Wasserstoff oder (C₁-C₆)-Alkyl.
S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan)
   (S13-5) der Firma Nitrokemia, das als Safener für Mais bekannt ist, "MG-838" (CAS-Reg.Nr. 133993-74-5)
   (2-propenyl 1-oxa-4-azaspiro[4.5]decane-4-carbodithioate) (S13-6) der Firma Nitrokemia
   "Disulfoton" (O,O-Diethyl S-2-ethylthioethyl phosphordithioat) (S13-7), "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist (S14-1),
   "Daimuron" oder "SK 23" (1-(1-Methyl-1-phenylethyl)-3-p-tolyl-harnstoff), das als Safener für Reis gegen Schäden des Herbizids Imazosulfuron bekannt ist (S14-2),
   "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist (S14-3),
   "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere wie sie in der WO-A-2008/131861 und WO-A-2008/131860 beschrieben sind 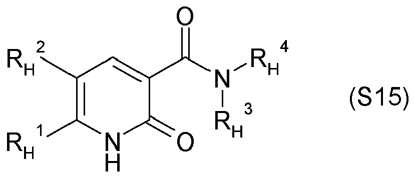 worin
   R_{H}¹ bedeutet einen (C₁-C₆)-Haloalkylrest,
   R_{H}² bedeutet Wasserstoff oder Halogen,
   R_{H}³, R_{H}⁴ bedeuten unabhängig voneinander Wasserstoff, (C₁-C₁₆)-Alkyl, (C₂-C₁₆)-Alkenyl oder (C₂-C₁₆)-Alkinyl, wobei jeder der letztgenannten 3 Reste unsubstituiert oder durch einen oder mehrere Reste aus der Gruppe Halogen, Hydroxy, Cyano, (C₁-C₄)-Alkoxy, (C₁-C₄)-Haloalkoxy, (C₁-C₄)-Alkylthio, (C₁-C₄)-Alkylamino, Di[(C₁-C₄)-alkyl]-amino, [(C₁-C₄)-Alkoxy]-carbonyl, [(C₁-C₄)-Haloalkoxy]-carbonyl, (C₃-C₆)-Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)-Cycloalkyl, (C₄-C₆)-Cycloalkenyl, (C₃-C₆)-Cycloalkyl, das an einer Seite des Rings mit einem 4 bis 6-gliedrigen gesättigten oder ungesättigten carbocyclischen Ring kondensiert ist, oder (C₄-C₆)-Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)-Alkyl, (C₁-C₄)-Haloalkyl, (C₁-C₄)-Alkoxy, (C₁-C₄)-Haloalkoxy, (C₁-C₄)-Alkylthio, (C₁-C₄)-Alkylamino, Di[(C₁-C₄)-alkyl]-amino, [(C₁-C₄)-Alkoxy]-carbonyl, [(C₁-C₄)-Haloalkoxy]-carbonyl, (C₃-C₆)-Cycloalkyl, das unsubstituiert oder substituiert ist, Phenyl, das unsubstituiert oder substituiert ist, und Heterocyclyl, das unsubstituiert oder substituiert ist, substituiert ist, oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)-Alkyl, (C₁-C₄)-Haloalkyl, (C₁-C₄)-Alkoxy, (C₁-C₄)-Haloalkoxy und (C₁-C₄)-Alkylthio substituiert ist.

Die zitierten Schriften enthalten ausführliche Angaben zu Herstellungsverfahren und Ausgangsmaterialien und nennen bevorzugte Verbindungen. Auf diese Schriften wird ausdrücklich Bezug genommen, sie gelten durch Zitat als Bestandteil dieser Beschreibung.

Eine besondere Bedeutung haben die Safener S1-1, S1-7, S1-11, S2-1, S3-1, S3-2, S3-4, S3-7, S3-8, S3-10, S3-11, S4-1, S4-5, S4-6, S11-1, S11-2, S11-3, S13-1, S13-2, S13-3, S13-8, S14-1, S14-2 und S14-3.

Beispiele für bevorzugte Zusammensetzungen von Herbiziden (A) und Safenern (B) sind in der folgenden Übersicht dargestellt:
(A1-1)+(S1-1), (A1-1)+(S1-7), (A1-1)+(S1-11), (A1-1)+(S2-1), (A1-1)+(S3-1), (A1-1)+(S3-2), (A1-1)+(S3-4), (A1-1)+(S3-7), (A1-1)+(S3-8), (A1-1)+(S3-10), (A1-1)+(S3-11), (A1-1)+(S4-1), (A1-1)+(S4-5), (A1-1)+(S4-6), (A1-1)+(S11-1), (A1-1)+(S11-2), (A1-1)+(S11-3), (A1-1)+(S13-1), (A1-1)+(S13-2), (A1-1)+(S13-3), (A1-1)+(S13-8), (A1-1)+(S14-1), (A1-1)+(S14-2), (A1-1)+(S14-3),
(A1-2)+(S1-1), (A1-2)+(S1-7), (A1-2)+(S1-11), (A1-2)+(S2-1), (A1-2)+(S3-1), (A1-2)+(S3-2), (A1-2)+(S3-4), (A1-2)+(S3-7), (A1-2)+(S3-8), (A1-2)+(S3-10), (A1-2)+(S3-11), (A1-2)+(S4-1), (A1-2)+(S4-5), (A1-2)+(S4-6), (A1-2)+(S11-1), (A1-2)+(S11-2), (A1-2)+(S11-3), (A1-2)+(S13-1), (A1-2)+(S13-2), (A1-2)+(S13-3), (A1-2)+(S13-8), (A1-2)+(S14-1), (A1-2)+(S14-2), (A1-2)+(S14-3),
(A1-3)+(S1-1), (A1-3)+(S1-7), (A1-3)+(S1-11), (A1-3)+(S2-1), (A1-3)+(S3-1), (A1-3)+(S3-2), (A1-3)+(S3-4), (A1-3)+(S3-7), (A1-3)+(S3-8), (A1-3)+(S3-10), (A1-3)+(S3-11), (A1-3)+(S4-1), (A1-3)+(S4-5), (A1-3)+(S4-6), (A1-3)+(S11-1), (A1-3)+(S11-2), (A1-3)+(S11-3), (A1-3)+(S13-1), (A1-3)+(S13-2), (A1-3)+(S13-3), (A1-3)+(S13-8), (A1-3)+(S14-1), (A1-3)+(S14-2), (A1-3)+(S14-3),
(A1-4)+(S1-1), (A1-4)+(S1-7), (A1-4)+(S1-11), (A1-4)+(S2-1), (A1-4)+(S3-1), (A1-4)+(S3-2), (A1-4)+(S3-4), (A1-4)+(S3-7), (A1-4)+(S3-8), (A1-4)+(S3-10), (A1-4)+(S3-11), (A1-4)+(S4-1), (A1-4)+(S4-5), (A1-4)+(S4-6), (A1-4)+(S11-1), (A1-4)+(S11-2), (A1-4)+(S11-3), (A1-4)+(S13-1), (A1-4)+(S13-2), (A1-4)+(S13-3), (A1-4)+(S13-8), (A1-4)+(S14-1), (A1-4)+(S14-2), (A1-4)+(S14-3),
(A1-5)+(S1-1), (A1-5)+(S1-7), (A1-5)+(S1-11), (A1-5)+(S2-1), (A1-5)+(S3-1), (A1-5)+(S3-2), (A1-5)+(S3-4), (A1-5)+(S3-7), (A1-5)+(S3-8), (A1-5)+(S3-10), (A1-5)+(S3-11), (A1-5)+(S4-1), (A1-5)+(S4-5), (A1-5)+(S4-6), (A1-5)+(S11-1), (A1-5)+(S11-2), (A1-5)+(S11-3), (A1-5)+(S13-1), (A1-5)+(S13-2), (A1-5)+(S13-3), (A1-5)+(S13-8), (A1-5)+(S14-1), (A1-5)+(S14-2), (A1-5)+(S14-3),
(A1-6)+(S1-1), (A1-6)+(S1-7), (A1-6)+(S1-11), (A1-6)+(S2-1), (A1-6)+(S3-1), (A1-6)+(S3-2), (A1-6)+(S3-4), (A1-6)+(S3-7), (A1-6)+(S3-8), (A1-6)+(S3-10), (A1-6)+(S3-11), (A1-6)+(S4-1), (A1-6)+(S4-5), (A1-6)+(S4-6), (A1-6)+(S11-1), (A1-6)+(S11-2), (A1-6)+(S11-3), (A1-6)+(S13-1), (A1-6)+(S13-2), (A1-6)+(S13-3), (A1-6)+(S13-8), (A1-6)+(S14-1), (A1-6)+(S14-2), (A1-6)+(S14-3),
(A1-7)+(S1-1), (A1-7)+(S1-7), (A1-7)+(S1-11), (A1-7)+(S2-1), (A1-7)+(S3-1), (A1-7)+(S3-2), (A1-7)+(S3-4), (A1-7)+(S3-7), (A1-7)+(S3-8), (A1-7)+(S3-10), (A1-7)+(S3-11), (A1-7)+(S4-1), (A1-7)+(S4-5), (A1-7)+(S4-6), (A1-7)+(S11-1), (A1-7)+(S11-2), (A1-7)+(S11-3), (A1-7)+(S13-1), (A1-7)+(S13-2), (A1-7)+(S13-3), (A1-7)+(S13-8), (A1-7)+(S14-1), (A1-7)+(S14-2), (A1-7)+(S14-3),
(A1-8)+(S1-1), (A1-8)+(S1-7), (A1-8)+(S1-11), (A1-8)+(S2-1), (A1-8)+(S3-1), (A1-8)+(S3-2), (A1-8)+(S3-4), (A1-8)+(S3-7), (A1-8)+(S3-8), (A1-8)+(S3-10), (A1-8)+(S3-11), (A1-8)+(S4-1), (A1-8)+(S4-5), (A1-8)+(S4-6), (A1-8)+(S11-1), (A1-8)+(S11-2), (A1-8)+(S11-3), (A1-8)+(S13-1), (A1-8)+(S13-2), (A1-8)+(S13-3), (A1-8)+(S13-8), (A1-8)+(S14-1), (A1-8)+(S14-2), (A1-8)+(S14-3),
(A1-9)+(S1-1), (A1-9)+(S1-7), (A1-9)+(S1-11), (A1-9)+(S2-1), (A1-9)+(S3-1), (A1-9)+(S3-2), (A1-9)+(S3-4), (A1-9)+(S3-7), (A1-9)+(S3-8), (A1-9)+(S3-10), (A1-9)+(S3-11), (A1-9)+(S4-1), (A1-9)+(S4-5), (A1-9)+(S4-6), (A1-9)+(S 11-1), (A1-9)+(S11-2), (A1-9)+(S11-3), (A1-9)+(S13-1), (A1-9)+(S13-2), (A1-9)+(S13-3), (A1-9)+(S13-8), (A1-9)+(S14-1), (A1-9)+(S14-2), (A1-9)+(S14-3), (A1-10)+(S1-1), (A1-10)+(S1-7), (A1-10)+(S1-11), (A1-10)+(S2-1), (A1-10)+(S3-1), (A1-10)+(S3-2), (A1-10)+(S3-4), (A1-10)+(S3-7), (A1-10)+(S3-8), (A1-10)+(S3-10), (A1-10)+(S3-11), (A1-10)+(S4-1), (A1-10)+(S4-5), (A1-10)+(S4-6), (A1-10)+(S11-1), (A1-10)+(S11-2), (A1-10)+(S11-3), (A1-10)+(S13-1), (A1-10)+(S13-2), (A1-10)+(S13-3), (A1-10)+(S13-8), (A1-10)+(S14-1), (A1-10)+(S14-2), (A1-10)+(S14-3),
(A1-11)+(S1-1), (A1-11)+(S1-7), (A1-11)+(S1-11), (A1-11)+(S2-1), (A1-11)+(S3-1), (A1-11)+(S3-2), (A1-11)+(S3-4), (A1-11)+(S3-7), (A1-11)+(S3-8), (A1-11)+(S3-10), (A1-11)+(S3-11), (A1-11)+(S4-1), (A1-11)+(S4-5), (A1-11)+(S4-6), (A1-11)+(S11-1), (A1-11)+(S11-2), (A1-11)+(S11-3), (A1-11)+(S13-1), (A1-11)+(S13-2), (A1-11)+(S13-3), (A1-11)+(S13-8), (A1-11)+(S14-1), (A1-11)+(S14-2), (A1-11)+(S14-3),
(A1-12)+(S1-1), (A1-12)+(S1-7), (A1-12)+(S1-11), (A1-12)+(S2-1), (A1-12)+(S3-1), (A1-12)+(S3-2), (A1-12)+(S3-4), (A1-12)+(S3-7), (A1-12)+(S3-8), (A1-12)+(S3-10), (A1-12)+(S3-11), (A1-12)+(S4-1), (A1-12)+(S4-5), (A1-12)+(S4-6), (A1-12)+(S11-1), (A1-12)+(S11-2), (A1-12)+(S11-3), (A1-12)+(S13-1), (A1-12)+(S13-2), (A1-12)+(S13-3), (A1-12)+(S13-8), (A1-12)+(S14-1), (A1-12)+(S14-2), (A1-12)+(S14-3),
(A1-13)+(S1-1), (A1-13)+(S1-7), (A1-13)+(S1-11), (A1-13)+(S2-1), (A1-13)+(S3-1), (A1-13)+(S3-2), (A1-13)+(S3-4), (A1-13)+(S3-7), (A1-13)+(S3-8), (A1-13)+(S3-10), (A1-13)+(S3-11), (A1-13)+(S4-1), (A1-13)+(S4-5), (A1-13)+(S4-6), (A1-13)+(S11-1), (A1-13)+(S11-2), (A1-13)+(S11-3), (A1-13)+(S13-1), (A1-13)+(S13-2), (A1-13)+(S13-3), (A1-13)+(S13-8), (A1-13)+(S14-1), (A1-13)+(S14-2), (A1-13)+(S14-3),
(A1-14)+(S1-1), (A1-14)+(S1-7), (A1-14)+(S1-11), (A1-14)+(S2-1), (A1-14)+(S3-1), (A1-14)+(S3-2), (A1-14)+(S3-4), (A1-14)+(S3-7), (A1-14)+(S3-8), (A1-14)+(S3-10), (A1-14)+(S3-11), (A1-14)+(S4-1), (A1-14)+(S4-5), (A1-14)+(S4-6), (A1-14)+(S11-1), (A1-14)+(S11-2), (A1-14)+(S11-3), (A1-14)+(S13-1), (A1-14)+(S13-2), (A1-14)+(S13-3), (A1-14)+(S13-8), (A1-14)+(S14-1), (A1-14)+(S14-2), (A1-14)+(S14-3),
(A1-15)+(S1-1), (A1-15)+(S1-7), (A1-15)+(S1-11), (A1-15)+(S2-1), (A1-15)+(S3-1), (A1-15)+(S3-2), (A1-15)+(S3-4), (A1-15)+(S3-7), (A1-15)+(S3-8), (A1-15)+(S3-10), (A1-15)+(S3-11), (A1-15)+(S4-1), (A1-15)+(S4-5), (A1-15)+(S4-6), (A1-15)+(S11-1), (A1-15)+(S11-2), (A1-15)+(S11-3), (A1-15)+(S13-1), (A1-15)+(S13-2), (A1-15)+(S13-3), (A1-15)+(S13-8), (A1-15)+(S14-1), (A1-15)+(S14-2), (A1-15)+(S14-3),
(A1-16)+(S1-1), (A1-16)+(S1-7), (A1-16)+(S1-11), (A1-16)+(S2-1), (A1-16)+(S3-1), (A1-16)+(S3-2), (A1-16)+(S3-4), (A1-16)+(S3-7), (A1-16)+(S3-8), (A1-16)+(S3-10), (A1-16)+(S3-11), (A1-16)+(S4-1), (A1-16)+(S4-5), (A1-16)+(S4-6), (A1-16)+(S11-1), (A1-16)+(S11-2), (A1-16)+(S11-3), (A1-16)+(S13-1), (A1-16)+(S13-2), (A1-16)+(S13-3), (A1-16)+(S13-8), (A1-16)+(S14-1), (A1-16)+(S14-2), (A1-16)+(S14-3),
(A1-17)+(S1-1), (A1-17)+(S1-7), (A1-17)+(S1-11), (A1-17)+(S2-1), (A1-17)+(S3-1), (A1-17)+(S3-2), (A1-17)+(S3-4), (A1-17)+(S3-7), (A1-17)+(S3-8), (A1-17)+(S3-10), (A1-17)+(S3-11), (A1-17)+(S4-1), (A1-17)+(S4-5), (A1-17)+(S4-6), (A1-17)+(S11-1), (A1-17)+(S11-2), (A1-17)+(S11-3), (A1-17)+(S13-1), (A1-17)+(S13-2), (A1-17)+(S13-3), (A1-17)+(S13-8), (A1-17)+(S14-1), (A1-17)+(S14-2), (A1-17)+(S14-3),
(A1-18)+(S1-1), (A1-18)+(S1-7), (A1-18)+(S1-11), (A1-18)+(S2-1), (A1-18)+(S3-1), (A1-18)+(S3-2), (A1-18)+(S3-4), (A1-18)+(S3-7), (A1-18)+(S3-8), (A1-18)+(S3-10), (A1-18)+(S3-11), (A1-18)+(S4-1), (A1-18)+(S4-5), (A1-18)+(S4-6), (A1-18)+(S11-1), (A1-18)+(S11-2), (A1-18)+(S11-3), (A1-18)+(S13-1), (A1-18)+(S13-2), (A1-18)+(S13-3), (A1-18)+(S13-8), (A1-18)+(S14-1), (A1-18)+(S14-2), (A1-18)+(S14-3),
(A1-19)+(S1-1), (A1-19)+(S1-7), (A1-19)+(S1-11), (A1-19)+(S2-1), (A1-19)+(S3-1), (A1-19)+(S3-2), (A1-19)+(S3-4), (A1-19)+(S3-7), (A1-19)+(S3-8), (A1-19)+(S3-10), (A1-19)+(S3-11), (A1-19)+(S4-1), (A1-19)+(S4-5), (A1-19)+(S4-6), (A1-19)+(S11-1), (A1-19)+(S11-2), (A1-19)+(S11-3), (A1-19)+(S13-1), (A1-19)+(S13-2), (A1-19)+(S13-3), (A1-19)+(S13-8), (A1-19)+(S14-1), (A1-19)+(S14-2), (A1-19)+(S14-3),
(A1-20)+(S1-1), (A1-20)+(S1-7), (A1-20)+(S1-11), (A1-20)+(S2-1), (A1-20)+(S3-1), (A1-20)+(S3-2), (A1-20)+(S3-4), (A1-20)+(S3-7), (A1-20)+(S3-8), (A1-20)+(S3-10), (A1-20)+(S3-11), (A1-20)+(S4-1), (A1-20)+(S4-5), (A1-20)+(S4-6), (A1-20)+(S11-1), (A1-20)+(S11-2), (A1-20)+(S11-3), (A1-20)+(S13-1), (A1-20)+(S13-2), (A1-20)+(S13-3), (A1-20)+(S13-8), (A1-20)+(S14-1), (A1-20)+(S14-2), (A1-20)+(S14-3),
(A1-21)+(S1-1), (A1-21)+(S1-7), (A1-21)+(S1-11), (A1-21)+(S2-1), (A1-21)+(S3-1), (A1-21)+(S3-2), (A1-21)+(S3-4), (A1-21)+(S3-7), (A1-21)+(S3-8), (A1-21)+(S3-10), (A1-21)+(S3-11), (A1-21)+(S4-1), (A1-21)+(S4-5), (A1-21)+(S4-6), (A1-21)+(S11-1), (A1-21)+(S11-2), (A1-21)+(S11-3), (A1-21)+(S13-1), (A1-21)+(S13-2), (A1-21)+(S13-3), (A1-21)+(S13-8), (A1-21)+(S14-1), (A1-21)+(S14-2), (A1-21)+(S14-3),
(A1-22)+(S1-1), (A1-22)+(S1-7), (A1-22)+(S1-11), (A1-22)+(S2-1), (A1-22)+(S3-1), (A1-22)+(S3-2), (A1-22)+(S3-4), (A1-22)+(S3-7), (A1-22)+(S3-8), (A1-22)+(S3-10), (A1-22)+(S3-11), (A1-22)+(S4-1), (A1-22)+(S4-5), (A1-22)+(S4-6), (A1-22)+(S11-1), (A1-22)+(S11-2), (A1-22)+(S11-3), (A1-22)+(S13-1), (A1-22)+(S13-2), (A1-22)+(S13-3), (A1-22)+(S13-8), (A1-22)+(S14-1), (A1-22)+(S14-2), (A1-22)+(S14-3),
(A1-23)+(S1-1), (A1-23)+(S1-7), (A1-23)+(S1-11), (A1-23)+(S2-1), (A1-23)+(S3-1), (A1-23)+(S3-2), (A1-23)+(S3-4), (A1-23)+(S3-7), (A1-23)+(S3-8), (A1-23)+(S3-10), (A1-23)+(S3-11), (A1-23)+(S4-1), (A1-23)+(S4-5), (A1-23)+(S4-6), (A1-23)+(S11-1), (A1-23)+(S11-2), (A1-23)+(S11-3), (A1-23)+(S13-1), (A1-23)+(S13-2), (A1-23)+(S13-3), (A1-23)+(S13-8), (A1-23)+(S14-1), (A1-23)+(S14-2), (A1-23)+(S14-3),
(A1-24)+(S1-1), (A1-24)+(S1-7), (A1-24)+(S1-11), (A1-24)+(S2-1), (A1-24)+(S3-1), (A1-24)+(S3-2), (A1-24)+(S3-4), (A1-24)+(S3-7), (A1-24)+(S3-8), (A1-24)+(S3-10), (A1-24)+(S3-11), (A1-24)+(S4-1), (A1-24)+(S4-5), (A1-24)+(S4-6), (A1-24)+(S11-1), (A1-24)+(S11-2), (A1-24)+(S11-3), (A1-24)+(S13-1), (A1-24)+(S13-2), (A1-24)+(S13-3), (A1-24)+(S13-8), (A1-24)+(S14-1), (A1-24)+(S14-2), (A1-24)+(S14-3),
(A1-25)+(S1-1), (A1-25)+(S1-7), (A1-25)+(S1-11), (A1-25)+(S2-1), (A1-25)+(S3-1), (A1-25)+(S3-2), (A1-25)+(S3-4), (A1-25)+(S3-7), (A1-25)+(S3-8), (A1-25)+(S3-10), (A1-25)+(S3-11), (A1-25)+(S4-1), (A1-25)+(S4-5), (A1-25)+(S4-6), (A1-25)+(S11-1), (A1-25)+(S11-2), (A1-25)+(S11-3), (A1-25)+(S13-1), (A1-25)+(S13-2), (A1-25)+(S13-3), (A1-25)+(S13-8), (A1-25)+(S14-1), (A1-25)+(S14-2), (A1-25)+(S14-3),
(A1-26)+(S1-1), (A1-26)+(S1-7), (A1-26)+(S1-11), (A1-26)+(S2-1), (A1-26)+(S3-1), (A1-26)+(S3-2), (A1-26)+(S3-4), (A1-26)+(S3-7), (A1-26)+(S3-8), (A1-26)+(S3-10), (A1-26)+(S3-11), (A1-26)+(S4-1), (A1-26)+(S4-5), (A1-26)+(S4-6), (A1-26)+(S11-1), (A1-26)+(S11-2), (A1-26)+(S11-3), (A1-26)+(S13-1), (A1-26)+(S13-2), (A1-26)+(S13-3), (A1-26)+(S13-8), (A1-26)+(S14-1), (A1-26)+(S14-2), (A1-26)+(S14-3),
(A1-27)+(S1-1), (A1-27)+(S1-7), (A1-27)+(S1-11), (A1-27)+(S2-1), (A1-27)+(S3-1), (A1-27)+(S3-2), (A1-27)+(S3-4), (A1-27)+(S3-7), (A1-27)+(S3-8), (A1-27)+(S3-10), (A1-27)+(S3-11), (A1-27)+(S4-1), (A1-27)+(S4-5), (A1-27)+(S4-6), (A1-27)+(S11-1), (A1-27)+(S11-2), (A1-27)+(S11-3), (A1-27)+(S13-1), (A1-27)+(S13-2), (A1-27)+(S13-3), (A1-27)+(S13-8), (A1-27)+(S14-1), (A1-27)+(S14-2), (A1-27)+(S14-3),
(A1-28)+(S1-1), (A1-28)+(S1-7), (A1-28)+(S1-11), (A1-28)+(S2-1), (A1-28)+(S3-1), (A1-28)+(S3-2), (A1-28)+(S3-4), (A1-28)+(S3-7), (A1-28)+(S3-8), (A1-28)+(S3-10), (A1-28)+(S3-11), (A1-28)+(S4-1), (A1-28)+(S4-5), (A1-28)+(S4-6), (A1-28)+(S11-1), (A1-28)+(S11-2), (A1-28)+(S11-3), (A1-28)+(S13-1), (A1-28)+(S13-2), (A1-28)+(S13-3), (A1-28)+(S13-8), (A1-28)+(S14-1), (A1-28)+(S14-2), (A1-28)+(S14-3),
(A1-29)+(S1-1), (A1-29)+(S1-7), (A1-29)+(S1-11), (A1-29)+(S2-1), (A1-29)+(S3-1), (A1-29)+(S3-2), (A1-29)+(S3-4), (A1-29)+(S3-7), (A1-29)+(S3-8), (A1-29)+(S3-10), (A1-29)+(S3-11), (A1-29)+(S4-1), (A1-29)+(S4-5), (A1-29)+(S4-6), (A1-29)+(S11-1), (A1-29)+(S11-2), (A1-29)+(S11-3), (A1-29)+(S13-1), (A1-29)+(S13-2), (A1-29)+(S13-3), (A1-29)+(S13-8), (A1-29)+(S14-1), (A1-29)+(S14-2), (A1-29)+(S14-3),
(A1-30)+(S1-1), (A1-30)+(S1-7), (A1-30)+(S1-11), (A1-30)+(S2-1), (A1-30)+(S3-1), (A1-30)+(S3-2), (A1-30)+(S3-4), (A1-30)+(S3-7), (A1-30)+(S3-8), (A1-30)+(S3-10), (A1-30)+(S3-11), (A1-30)+(S4-1), (A1-30)+(S4-5), (A1-30)+(S4-6), (A1-30)+(S11-1), (A1-30)+(S11-2), (A1-30)+(S11-3), (A1-30)+(S13-1), (A1-30)+(S13-2), (A1-30)+(S13-3), (A1-30)+(S13-8), (A1-30)+(S14-1), (A1-30)+(S14-2), (A1-30)+(S14-3),
(A1-31)+(S1-1), (A1-31)+(S1-7), (A1-31)+(S1-11), (A1-31)+(S2-1), (A1-31)+(S3-1), (A1-31)+(S3-2), (A1-31)+(S3-4), (A1-31)+(S3-7), (A1-31)+(S3-8), (A1-31)+(S3-10), (A1-31)+(S3-11), (A1-31)+(S4-1), (A1-31)+(S4-5), (A1-31)+(S4-6), (A1-31)+(S11-1), (A1-31)+(S11-2), (A1-31)+(S11-3), (A1-31)+(S13-1), (A1-31)+(S13-2), (A1-31)+(S13-3), (A1-31)+(S13-8), (A1-31)+(S14-1), (A1-31)+(S14-2), (A1-31)+(S14-3),
(A1-32)+(S1-1), (A1-32)+(S1-7), (A1-32)+(S1-11), (A1-32)+(S2-1), (A1-32)+(S3-1), (A1-32)+(S3-2), (A1-32)+(S3-4), (A1-32)+(S3-7), (A1-32)+(S3-8), (A1-32)+(S3-10), (A1-32)+(S3-11), (A1-32)+(S4-1), (A1-32)+(S4-5), (A1-32)+(S4-6), (A1-32)+(S11-1), (A1-32)+(S11-2), (A1-32)+(S11-3), (A1-32)+(S13-1), (A1-32)+(S13-2), (A1-32)+(S13-3), (A1-32)+(S13-8), (A1-32)+(S14-1), (A1-32)+(S14-2), (A1-32)+(S14-3),
(A1-33)+(S1-1), (A1-33)+(S1-7), (A1-33)+(S1-11), (A1-33)+(S2-1), (A1-33)+(S3-1), (A1-33)+(S3-2), (A1-33)+(S3-4), (A1-33)+(S3-7), (A1-33)+(S3-8), (A1-33)+(S3-10), (A1-33)+(S3-11), (A1-33)+(S4-1), (A1-33)+(S4-5), (A1-33)+(S4-6), (A1-33)+(S11-1), (A1-33)+(S11-2), (A1-33)+(S11-3), (A1-33)+(S13-1), (A1-33)+(S13-2), (A1-33)+(S13-3), (A1-33)+(S13-8), (A1-33)+(S14-1), (A1-33)+(S14-2), (A1-33)+(S14-3),
(A1-34)+(S1-1), (A1-34)+(S1-7), (A1-34)+(S1-11), (A1-34)+(S2-1), (A1-34)+(S3-1), (A1-34)+(S3-2), (A1-34)+(S3-4), (A1-34)+(S3-7), (A1-34)+(S3-8), (A1-34)+(S3-10), (A1-34)+(S3-11), (A1-34)+(S4-1), (A1-34)+(S4-5), (A1-34)+(S4-6), (A1-34)+(S11-1), (A1-34)+(S11-2), (A1-34)+(S11-3), (A1-34)+(S13-1), (A1-34)+(S13-2), (A1-34)+(S13-3), (A1-34)+(S13-8), (A1-34)+(S14-1), (A1-34)+(S14-2), (A1-34)+(S14-3),
(A1-35)+(S1-1), (A1-35)+(S1-7), (A1-35)+(S1-11), (A1-35)+(S2-1), (A1-35)+(S3-1), (A1-35)+(S3-2), (A1-35)+(S3-4), (A1-35)+(S3-7), (A1-35)+(S3-8), (A1-35)+(S3-10), (A1-35)+(S3-11), (A1-35)+(S4-1), (A1-35)+(S4-5), (A1-35)+(S4-6), (A1-35)+(S11-1), (A1-35)+(S11-2), (A1-35)+(S11-3), (A1-35)+(S13-1), (A1-35)+(S13-2), (A1-35)+(S13-3), (A1-35)+(S13-8), (A1-35)+(S14-1), (A1-35)+(S14-2), (A1-35)+(S14-3),
(A1-36)+(S1-1), (A1-36)+(S1-7), (A1-36)+(S1-11), (A1-36)+(S2-1), (A1-36)+(S3-1), (A1-36)+(S3-2), (A1-36)+(S3-4), (A1-36)+(S3-7), (A1-36)+(S3-8), (A1-36)+(S3-10), (A1-36)+(S3-11), (A1-36)+(S4-1), (A1-36)+(S4-5), (A1-36)+(S4-6), (A1-36)+(S11-1), (A1-36)+(S11-2), (A1-36)+(S11-3), (A1-36)+(S13-1), (A1-36)+(S13-2), (A1-36)+(S13-3), (A1-36)+(S13-8), (A1-36)+(S14-1), (A1-36)+(S14-2), (A1-36)+(S14-3),
(A1-37)+(S1-1), (A1-37)+(S1-7), (A1-37)+(S1-11), (A1-37)+(S2-1), (A1-37)+(S3-1), (A1-37)+(S3-2), (A1-37)+(S3-4), (A1-37)+(S3-7), (A1-37)+(S3-8), (A1-37)+(S3-10), (A1-37)+(S3-11), (A1-37)+(S4-1), (A1-37)+(S4-5), (A1-37)+(S4-6), (A1-37)+(S11-1), (A1-37)+(S11-2), (A1-37)+(S11-3), (A1-37)+(S13-1), (A1-37)+(S13-2), (A1-37)+(S13-3), (A1-37)+(S13-8), (A1-37)+(S14-1), (A1-37)+(S14-2), (A1-37)+(S14-3),
(A1-38)+(S1-1), (A1-38)+(S1-7), (A1-38)+(S1-11), (A1-38)+(S2-1), (A1-38)+(S3-1), (A1-38)+(S3-2), (A1-38)+(S3-4), (A1-38)+(S3-7), (A1-38)+(S3-8), (A1-38)+(S3-10), (A1-38)+(S3-11), (A1-38)+(S4-1), (A1-38)+(S4-5), (A1-38)+(S4-6), (A1-38)+(S11-1), (A1-38)+(S11-2), (A1-38)+(S11-3), (A1-38)+(S13-1), (A1-38)+(S13-2), (A1-38)+(S13-3), (A1-38)+(S13-8), (A1-38)+(S14-1), (A1-38)+(S14-2), (A1-38)+(S14-3),
(A1-39)+(S1-1), (A1-39)+(S1-7), (A1-39)+(S1-11), (A1-39)+(S2-1), (A1-39)+(S3-1), (A1-39)+(S3-2), (A1-39)+(S3-4), (A1-39)+(S3-7), (A1-39)+(S3-8), (A1-39)+(S3-10), (A1-39)+(S3-11), (A1-39)+(S4-1), (A1-39)+(S4-5), (A1-39)+(S4-6), (A1-39)+(S1-1), (A1-39)+(S11-2), (A1-39)+(S11-3), (A1-39)+(S13-1), (A1-39)+(S13-2), (A1-39)+(S13-3), (A1-39)+(S13-8), (A1-39)+(S14-1), (A1-39)+(S14-2), (A1-39)+(S14-3),
(A1-40)+(S1-1), (A1-40)+(S1-7), (A1-40)+(S1-11), (A1-40)+(S2-1), (A1-40)+(S3-1), (A1-40)+(S3-2), (A1-40)+(S3-4), (A1-40)+(S3-7), (A1-40)+(S3-8), (A1-40)+(S3-10), (A1-40)+(S3-11), (A1-40)+(S4-1), (A1-40)+(S4-5), (A1-40)+(S4-6), (A1-40)+(S1-1), (A1-40)+(S11-2), (A1-40)+(S11-3), (A1-40)+(S13-1), (A1-40)+(S13-2), (A1-40)+(S13-3), (A1-40)+(S13-8), (A1-40)+(S14-1), (A1-40)+(S14-2), (A1-40)+(S14-3),
(A1-41)+(S1-1), (A1-41)+(S1-7), (A1-41)+(S1-11), (A1-41)+(S2-1), (A1-41)+(S3-1), (A1-41)+(S3-2), (A1-41)+(S3-4), (A1-41)+(S3-7), (A1-41)+(S3-8), (A1-41)+(S3-10), (A1-41)+(S3-11), (A1-41)+(S4-1), (A1-41)+(S4-5), (A1-41)+(S4-6), (A1-41)+(S1-1), (A1-41)+(S11-2), (A1-41)+(S11-3), (A1-41)+(S13-1), (A1-41)+(S13-2), (A1-41)+(S13-3), (A1-41)+(S13-8), (A1-41)+(S14-1), (A1-41)+(S14-2), (A1-41)+(S14-3),
(A1-42)+(S1-1), (A1-42)+(S1-7), (A1-42)+(S1-11), (A1-42)+(S2-1), (A1-42)+(S3-1), (A1-42)+(S3-2), (A1-42)+(S3-4), (A1-42)+(S3-7), (A1-42)+(S3-8), (A1-42)+(S3-10), (A1-42)+(S3-11), (A1-42)+(S4-1), (A1-42)+(S4-5), (A1-42)+(S4-6), (A1-42)+(S11-1), (A1-42)+(S11-2), (A1-42)+(S11-3), (A1-42)+(S13-1), (A1-42)+(S13-2), (A1-42)+(S13-3), (A1-42)+(S13-8), (A1-42)+(S14-1), (A1-42)+(S14-2), (A1-42)+(S14-3),
(A1-43)+(S1-1), (A1-43)+(S1-7), (A1-43)+(S1-11), (A1-43)+(S2-1), (A1-43)+(S3-1), (A1-43)+(S3-2), (A1-43)+(S3-4), (A1-43)+(S3-7), (A1-43)+(S3-8), (A1-43)+(S3-10), (A1-43)+(S3-11), (A1-43)+(S4-1), (A1-43)+(S4-5), (A1-43)+(S4-6), (A1-43)+(S11-1), (A1-43)+(S11-2), (A1-43)+(S11-3), (A1-43)+(S13-1), (A1-43)+(S13-2), (A1-43)+(S13-3), (A1-43)+(S13-8), (A1-43)+(S14-1), (A1-43)+(S14-2), (A1-43)+(S14-3),
(A1-44)+(S1-1), (A1-44)+(S1-7), (A1-44)+(S1-11), (A1-44)+(S2-1), (A1-44)+(S3-1), (A1-44)+(S3-2), (A1-44)+(S3-4), (A1-44)+(S3-7), (A1-44)+(S3-8), (A1-44)+(S3-10), (A1-44)+(S3-11), (A1-44)+(S4-1), (A1-44)+(S4-5), (A1-44)+(S4-6), (A1-44)+(S11-1), (A1-44)+(S11-2), (A1-44)+(S11-3), (A1-44)+(S13-1), (A1-44)+(S13-2), (A1-44)+(S13-3), (A1-44)+(S13-8), (A1-44)+(S14-1), (A1-44)+(S14-2), (A1-44)+(S14-3),
(A1-45)+(S1-1), (A1-45)+(S1-7), (A1-45)+(S1-11), (A1-45)+(S2-1), (A1-45)+(S3-1), (A1-45)+(S3-2), (A1-45)+(S3-4), (A1-45)+(S3-7), (A1-45)+(S3-8), (A1-45)+(S3-10), (A1-45)+(S3-11), (A1-45)+(S4-1), (A1-45)+(S4-5), (A1-45)+(S4-6), (A1-45)+(S11-1), (A1-45)+(S11-2), (A1-45)+(S11-3), (A1-45)+(S13-1), (A1-45)+(S13-2), (A1-45)+(S13-3), (A1-45)+(S13-8), (A1-45)+(S14-1), (A1-45)+(S14-2), (A1-45)+(S14-3),
(A1-46)+(S1-1), (A1-46)+(S1-7), (A1-46)+(S1-11), (A1-46)+(S2-1), (A1-46)+(S3-1), (A1-46)+(S3-2), (A1-46)+(S3-4), (A1-46)+(S3-7), (A1-46)+(S3-8), (A1-46)+(S3-10), (A1-46)+(S3-11), (A1-46)+(S4-1), (A1-46)+(S4-5), (A1-46)+(S4-6), (A1-46)+(S11-1), (A1-46)+(S11-2), (A1-46)+(S11-3), (A1-46)+(S13-1), (A1-46)+(S13-2), (A1-46)+(S13-3), (A1-46)+(S13-8), (A1-46)+(S14-1), (A1-46)+(S14-2), (A1-46)+(S14-3),
(A1-47)+(S1-1), (A1-47)+(S1-7), (A1-47)+(S1-11), (A1-47)+(S2-1), (A1-47)+(S3-1), (A1-47)+(S3-2), (A1-47)+(S3-4), (A1-47)+(S3-7), (A1-47)+(S3-8), (A1-47)+(S3-10), (A1-47)+(S3-11), (A1-47)+(S4-1), (A1-47)+(S4-5), (A1-47)+(S4-6), (A1-47)+(S11-1), (A1-47)+(S11-2), (A1-47)+(S11-3), (A1-47)+(S13-1), (A1-47)+(S13-2), (A1-47)+(S13-3), (A1-47)+(S13-8), (A1-47)+(S14-1), (A1-47)+(S14-2), (A1-47)+(S14-3),
(A1-48)+(S1-1), (A1-48)+(S1-7), (A1-48)+(S1-11), (A1-48)+(S2-1), (A1-48)+(S3-1), (A1-48)+(S3-2), (A1-48)+(S3-4), (A1-48)+(S3-7), (A1-48)+(S3-8), (A1-48)+(S3-10), (A1-48)+(S3-11), (A1-48)+(S4-1), (A1-48)+(S4-5), (A1-48)+(S4-6), (A1-48)+(S11-1), (A1-48)+(S11-2), (A1-48)+(S11-3), (A1-48)+(S13-1), (A1-48)+(S13-2), (A1-48)+(S13-3), (A1-48)+(S13-8), (A1-48)+(S14-1), (A1-48)+(S14-2), (A1-48)+(S14-3),
(A1-49)+(S1-1), (A1-49)+(S1-7), (A1-49)+(S1-11), (A1-49)+(S2-1), (A1-49)+(S3-1), (A1-49)+(S3-2), (A1-49)+(S3-4), (A1-49)+(S3-7), (A1-49)+(S3-8), (A1-49)+(S3-10), (A1-49)+(S3-11), (A1-49)+(S4-1), (A1-49)+(S4-5), (A1-49)+(S4-6), (A1-49)+(S11-1), (A1-49)+(S11-2), (A1-49)+(S11-3), (A1-49)+(S13-1), (A1-49)+(S13-2), (A1-49)+(S13-3), (A1-49)+(S13-8), (A1-49)+(S14-1), (A1-49)+(S14-2), (A1-49)+(S14-3),
(A1-50)+(S1-1), (A1-50)+(S1-7), (A1-50)+(S1-11), (A1-50)+(S2-1), (A1-50)+(S3-1), (A1-50)+(S3-2), (A1-50)+(S3-4), (A1-50)+(S3-7), (A1-50)+(S3-8), (A1-50)+(S3-10), (A1-50)+(S3-11), (A1-50)+(S4-1), (A1-50)+(S4-5), (A1-50)+(S4-6), (A1-50)+(S11-1), (A1-50)+(S11-2), (A1-50)+(S11-3), (A1-50)+(S13-1), (A1-50)+(S13-2), (A1-50)+(S13-3), (A1-50)+(S13-8), (A1-50)+(S14-1), (A1-50)+(S14-2), (A1-50)+(S14-3),
(A1-51)+(S1-1), (A1-51)+(S1-7), (A1-51)+(S1-11), (A1-51)+(S2-1), (A1-51)+(S3-1), (A1-51)+(S3-2), (A1-51)+(S3-4), (A1-51)+(S3-7), (A1-51)+(S3-8), (A1-51)+(S3-10), (A1-51)+(S3-11), (A1-51)+(S4-1), (A1-51)+(S4-5), (A1-51)+(S4-6), (A1-51)+(S11-1), (A1-51)+(S11-2), (A1-51)+(S11-3), (A1-51)+(S13-1), (A1-51)+(S13-2), (A1-51)+(S13-3), (A1-51)+(S13-8), (A1-51)+(S14-1), (A1-51)+(S14-2), (A1-51)+(S14-3),
(A1-52)+(S1-1), (A1-52)+(S1-7), (A1-52)+(S1-11), (A1-52)+(S2-1), (A1-52)+(S3-1), (A1-52)+(S3-2), (A1-52)+(S3-4), (A1-52)+(S3-7), (A1-52)+(S3-8), (A1-52)+(S3-10), (A1-52)+(S3-11), (A1-52)+(S4-1), (A1-52)+(S4-5), (A1-52)+(S4-6), (A1-52)+(S11-1), (A1-52)+(S11-2), (A1-52)+(S11-3), (A1-52)+(S13-1), (A1-52)+(S13-2), (A1-52)+(S13-3), (A1-52)+(S13-8), (A1-52)+(S14-1), (A1-52)+(S14-2), (A1-52)+(S14-3),
(A1-53)+(S1-1), (A1-53)+(S1-7), (A1-53)+(S1-11), (A1-53)+(S2-1), (A1-53)+(S3-1), (A1-53)+(S3-2), (A1-53)+(S3-4), (A1-53)+(S3-7), (A1-53)+(S3-8), (A1-53)+(S3-10), (A1-53)+(S3-11), (A1-53)+(S4-1), (A1-53)+(S4-5), (A1-53)+(S4-6), (A1-53)+(S1-1), (A1-53)+(S11-2), (A1-53)+(S11-3), (A1-53)+(S13-1), (A1-53)+(S13-2), (A1-53)+(S13-3), (A1-53)+(S13-8), (A1-53)+(S14-1), (A1-53)+(S14-2), (A1-53)+(S14-3),
(A1-54)+(S1-1), (A1-54)+(S1-7), (A1-54)+(S1-11), (A1-54)+(S2-1), (A1-54)+(S3-1), (A1-54)+(S3-2), (A1-54)+(S3-4), (A1-54)+(S3-7), (A1-54)+(S3-8), (A1-54)+(S3-10), (A1-54)+(S3-11), (A1-54)+(S4-1), (A1-54)+(S4-5), (A1-54)+(S4-6), (A1-54)+(S11-1), (A1-54)+(S11-2), (A1-54)+(S11-3), (A1-54)+(S13-1), (A1-54)+(S13-2), (A1-54)+(S13-3), (A1-54)+(S13-8), (A1-54)+(S14-1), (A1-54)+(S14-2), (A1-54)+(S14-3),
(A1-55)+(S1-1), (A1-55)+(S1-7), (A1-55)+(S1-11), (A1-55)+(S2-1), (A1-55)+(S3-1), (A1-55)+(S3-2), (A1-55)+(S3-4), (A1-55)+(S3-7), (A1-55)+(S3-8), (A1-55)+(S3-10), (A1-55)+(S3-11), (A1-55)+(S4-1), (A1-55)+(S4-5), (A1-55)+(S4-6), (A1-55)+(S11-1), (A1-55)+(S11-2), (A1-55)+(S11-3), (A1-55)+(S13-1), (A1-55)+(S13-2), (A1-55)+(S13-3), (A1-55)+(S13-8), (A1-55)+(S14-1), (A1-55)+(S14-2), (A1-55)+(S14-3),
(A1-56)+(S1-1), (A1-56)+(S1-7), (A1-56)+(S1-11), (A1-56)+(S2-1), (A1-56)+(S3-1), (A1-56)+(S3-2), (A1-56)+(S3-4), (A1-56)+(S3-7), (A1-56)+(S3-8), (A1-56)+(S3-10), (A1-56)+(S3-11), (A1-56)+(S4-1), (A1-56)+(S4-5), (A1-56)+(S4-6), (A1-56)+(S11-1), (A1-56)+(S11-2), (A1-56)+(S11-3), (A1-56)+(S13-1), (A1-56)+(S13-2), (A1-56)+(S13-3), (A1-56)+(S13-8), (A1-56)+(S14-1), (A1-56)+(S14-2), (A1-56)+(S14-3),
(A1-57)+(S1-1), (A1-57)+(S1-7), (A1-57)+(S1-11), (A1-57)+(S2-1), (A1-57)+(S3-1), (A1-57)+(S3-2), (A1-57)+(S3-4), (A1-57)+(S3-7), (A1-57)+(S3-8), (A1-57)+(S3-10), (A1-57)+(S3-11), (A1-57)+(S4-1), (A1-57)+(S4-5), (A1-57)+(S4-6), (A1-57)+(S11-1), (A1-57)+(S11-2), (A1-57)+(S11-3), (A1-57)+(S13-1), (A1-57)+(S13-2), (A1-57)+(S13-3), (A1-57)+(S13-8), (A1-57)+(S14-1), (A1-57)+(S14-2), (A1-57)+(S14-3),
(A1-58)+(S1-1), (A1-58)+(S1-7), (A1-58)+(S1-11), (A1-58)+(S2-1), (A1-58)+(S3-1), (A1-58)+(S3-2), (A1-58)+(S3-4), (A1-58)+(S3-7), (A1-58)+(S3-8), (A1-58)+(S3-10), (A1-58)+(S3-11), (A1-58)+(S4-1), (A1-58)+(S4-5), (A1-58)+(S4-6), (A1-58)+(S11-1), (A1-58)+(S11-2), (A1-58)+(S11-3), (A1-58)+(S13-1), (A1-58)+(S13-2), (A1-58)+(S13-3), (A1-58)+(S13-8), (A1-58)+(S14-1), (A1-58)+(S14-2), (A1-58)+(S14-3),
(A1-59)+(S1-1), (A1-59)+(S1-7), (A1-59)+(S1-11), (A1-59)+(S2-1), (A1-59)+(S3-1), (A1-59)+(S3-2), (A1-59)+(S3-4), (A1-59)+(S3-7), (A1-59)+(S3-8), (A1-59)+(S3-10), (A1-59)+(S3-11), (A1-59)+(S4-1), (A1-59)+(S4-5), (A1-59)+(S4-6), (A1-59)+(S11-1), (A1-59)+(S11-2), (A1-59)+(S11-3), (A1-59)+(S13-1), (A1-59)+(S13-2), (A1-59)+(S13-3), (A1-59)+(S13-8), (A1-59)+(S14-1), (A1-59)+(S14-2), (A1-59)+(S14-3),
(A1-60)+(S1-1), (A1-60)+(S1-7), (A1-60)+(S1-11), (A1-60)+(S2-1), (A1-60)+(S3-1), (A1-60)+(S3-2), (A1-60)+(S3-4), (A1-60)+(S3-7), (A1-60)+(S3-8), (A1-60)+(S3-10), (A1-60)+(S3-11), (A1-60)+(S4-1), (A1-60)+(S4-5), (A1-60)+(S4-6), (A1-60)+(S11-1), (A1-60)+(S11-2), (A1-60)+(S11-3), (A1-60)+(S13-1), (A1-60)+(S13-2), (A1-60)+(S13-3), (A1-60)+(S13-8), (A1-60)+(S14-1), (A1-60)+(S14-2), (A1-60)+(S14-3),
(A1-61)+(S1-1), (A1-61)+(S1-7), (A1-61)+(S1-11), (A1-61)+(S2-1), (A1-61)+(S3-1), (A1-61)+(S3-2), (A1-61)+(S3-4), (A1-61)+(S3-7), (A1-61)+(S3-8), (A1-61)+(S3-10), (A1-61)+(S3-11), (A1-61)+(S4-1), (A1-61)+(S4-5), (A1-61)+(S4-6), (A1-61)+(S11-1), (A1-61)+(S11-2), (A1-61)+(S11-3), (A1-61)+(S13-1), (A1-61)+(S13-2), (A1-61)+(S13-3), (A1-61)+(S13-8), (A1-61)+(S14-1), (A1-61)+(S14-2), (A1-61)+(S14-3),
(A1-62)+(S1-1), (A1-62)+(S1-7), (A1-62)+(S1-11), (A1-62)+(S2-1), (A1-62)+(S3-1), (A1-62)+(S3-2), (A1-62)+(S3-4), (A1-62)+(S3-7), (A1-62)+(S3-8), (A1-62)+(S3-10), (A1-62)+(S3-11), (A1-62)+(S4-1), (A1-62)+(S4-5), (A1-62)+(S4-6), (A1-62)+(S11-1), (A1-62)+(S11-2), (A1-62)+(S11-3), (A1-62)+(S13-1), (A1-62)+(S13-2), (A1-62)+(S13-3), (A1-62)+(S13-8), (A1-62)+(S14-1), (A1-62)+(S14-2), (A1-62)+(S14-3),
(A1-63)+(S1-1), (A1-63)+(S1-7), (A1-63)+(S1-11), (A1-63)+(S2-1), (A1-63)+(S3-1), (A1-63)+(S3-2), (A1-63)+(S3-4), (A1-63)+(S3-7), (A1-63)+(S3-8), (A1-63)+(S3-10), (A1-63)+(S3-11), (A1-63)+(S4-1), (A1-63)+(S4-5), (A1-63)+(S4-6), (A1-63)+(S11-1), (A1-63)+(S11-2), (A1-63)+(S11-3), (A1-63)+(S13-1), (A1-63)+(S13-2), (A1-63)+(S13-3), (A1-63)+(S13-8), (A1-63)+(S14-1), (A1-63)+(S14-2), (A1-63)+(S14-3), (A1-64)+(S1-1), (A1-64)+(S1-7), (A1-64)+(S1-11), (A1-64)+(S2-1), (A1-64)+(S3-1), (A1-64)+(S3-2), (A1-64)+(S3-4), (A1-64)+(S3-7), (A1-64)+(S3-8), (A1-64)+(S3-10), (A1-64)+(S3-11), (A1-64)+(S4-1), (A1-64)+(S4-5), (A1-64)+(S4-6), (A1-64)+(S11-1), (A1-64)+(S11-2), (A1-64)+(S11-3), (A1-64)+(S13-1), (A1-64)+(S13-2), (A1-64)+(S13-3), (A1-64)+(S13-8), (A1-64)+(S14-1), (A1-64)+(S14-2), (A1-64)+(S14-3),
(A1-65)+(S1-1), (A1-65)+(S1-7), (A1-65)+(S1-11), (A1-65)+(S2-1), (A1-65)+(S3-1), (A1-65)+(S3-2), (A1-65)+(S3-4), (A1-65)+(S3-7), (A1-65)+(S3-8), (A1-65)+(S3-10), (A1-65)+(S3-11), (A1-65)+(S4-1), (A1-65)+(S4-5), (A1-65)+(S4-6), (A1-65)+(S11-1), (A1-65)+(S11-2), (A1-65)+(S11-3), (A1-65)+(S13-1), (A1-65)+(S13-2), (A1-65)+(S13-3), (A1-65)+(S13-8), (A1-65)+(S14-1), (A1-65)+(S14-2), (A1-65)+(S14-3),
(A1-66)+(S1-1), (A1-66)+(S1-7), (A1-66)+(S1-11), (A1-66)+(S2-1), (A1-66)+(S3-1), (A1-66)+(S3-2), (A1-66)+(S3-4), (A1-66)+(S3-7), (A1-66)+(S3-8), (A1-66)+(S3-10), (A1-66)+(S3-11), (A1-66)+(S4-1), (A1-66)+(S4-5), (A1-66)+(S4-6), (A1-66)+(S11-1), (A1-66)+(S11-2), (A1-66)+(S11-3), (A1-66)+(S13-1), (A1-66)+(S13-2), (A1-66)+(S13-3), (A1-66)+(S13-8), (A1-66)+(S14-1), (A1-66)+(S14-2), (A1-66)+(S14-3),
(A1-67)+(S1-1), (A1-67)+(S1-7), (A1-67)+(S1-11), (A1-67)+(S2-1), (A1-67)+(S3-1), (A1-67)+(S3-2), (A1-67)+(S3-4), (A1-67)+(S3-7), (A1-67)+(S3-8), (A1-67)+(S3-10), (A1-67)+(S3-11), (A1-67)+(S4-1), (A1-67)+(S4-5), (A1-67)+(S4-6), (A1-67)+(S11-1), (A1-67)+(S11-2), (A1-67)+(S11-3), (A1-67)+(S13-1), (A1-67)+(S13-2), (A1-67)+(S13-3), (A1-67)+(S13-8), (A1-67)+(S14-1), (A1-67)+(S14-2), (A1-67)+(S14-3),
(A1-68)+(S1-1), (A1-68)+(S1-7), (A1-68)+(S1-11), (A1-68)+(S2-1), (A1-68)+(S3-1), (A1-68)+(S3-2), (A1-68)+(S3-4), (A1-68)+(S3-7), (A1-68)+(S3-8), (A1-68)+(S3-10), (A1-68)+(S3-11), (A1-68)+(S4-1), (A1-68)+(S4-5), (A1-68)+(S4-6), (A1-68)+(S11-1), (A1-68)+(S11-2), (A1-68)+(S11-3), (A1-68)+(S13-1), (A1-68)+(S13-2), (A1-68)+(S13-3), (A1-68)+(S13-8), (A1-68)+(S14-1), (A1-68)+(S14-2), (A1-68)+(S14-3),
(A1-69)+(S1-1), (A1-69)+(S1-7), (A1-69)+(S1-11), (A1-69)+(S2-1), (A1-69)+(S3-1), (A1-69)+(S3-2), (A1-69)+(S3-4), (A1-69)+(S3-7), (A1-69)+(S3-8), (A1-69)+(S3-10), (A1-69)+(S3-11), (A1-69)+(S4-1), (A1-69)+(S4-5), (A1-69)+(S4-6), (A1-69)+(S11-1), (A1-69)+(S11-2), (A1-69)+(S11-3), (A1-69)+(S13-1), (A1-69)+(S13-2), (A1-69)+(S13-3), (A1-69)+(S13-8), (A1-69)+(S14-1), (A1-69)+(S14-2), (A1-69)+(S14-3),
(A1-70)+(S1-1), (A1-70)+(S1-7), (A1-70)+(S1-11), (A1-70)+(S2-1), (A1-70)+(S3-1), (A1-70)+(S3-2), (A1-70)+(S3-4), (A1-70)+(S3-7), (A1-70)+(S3-8), (A1-70)+(S3-10), (A1-70)+(S3-11), (A1-70)+(S4-1), (A1-70)+(S4-5), (A1-70)+(S4-6), (A1-70)+(S11-1), (A1-70)+(S11-2), (A1-70)+(S11-3), (A1-70)+(S13-1), (A1-70)+(S13-2), (A1-70)+(S13-3), (A1-70)+(S13-8), (A1-70)+(S14-1), (A1-70)+(S14-2), (A1-70)+(S14-3),
(A1-71)+(S1-1), (A1-71)+(S1-7), (A1-71)+(S1-11), (A1-71)+(S2-1), (A1-71)+(S3-1), (A1-71)+(S3-2), (A1-71)+(S3-4), (A1-71)+(S3-7), (A1-71)+(S3-8), (A1-71)+(S3-10), (A1-71)+(S3-11), (A1-71)+(S4-1), (A1-71)+(S4-5), (A1-71)+(S4-6), (A1-71)+(S11-1), (A1-71)+(S11-2), (A1-71)+(S11-3), (A1-71)+(S13-1), (A1-71)+(S13-2), (A1-71)+(S13-3), (A1-71)+(S13-8), (A1-71)+(S14-1), (A1-71)+(S14-2), (A1-71)+(S14-3),
(A1-72)+(S1-1), (A1-72)+(S1-7), (A1-72)+(S1-11), (A1-72)+(S2-1), (A1-72)+(S3-1), (A1-72)+(S3-2), (A1-72)+(S3-4), (A1-72)+(S3-7), (A1-72)+(S3-8), (A1-72)+(S3-10), (A1-72)+(S3-11), (A1-72)+(S4-1), (A1-72)+(S4-5), (A1-72)+(S4-6), (A1-72)+(S11-1), (A1-72)+(S11-2), (A1-72)+(S11-3), (A1-72)+(S13-1), (A1-72)+(S13-2), (A1-72)+(S13-3), (A1-72)+(S13-8), (A1-72)+(S14-1), (A1-72)+(S14-2), (A1-72)+(S14-3),
(A1-73)+(S1-1), (A1-73)+(S1-7), (A1-73)+(S1-11), (A1-73)+(S2-1), (A1-73)+(S3-1), (A1-73)+(S3-2), (A1-73)+(S3-4), (A1-73)+(S3-7), (A1-73)+(S3-8), (A1-73)+(S3-10), (A1-73)+(S3-11), (A1-73)+(S4-1), (A1-73)+(S4-5), (A1-73)+(S4-6), (A1-73)+(S11-1), (A1-73)+(S11-2), (A1-73)+(S11-3), (A1-73)+(S13-1), (A1-73)+(S13-2), (A1-73)+(S13-3), (A1-73)+(S13-8), (A1-73)+(S14-1), (A1-73)+(S14-2), (A1-73)+(S14-3),
(A1-74)+(S1-1), (A1-74)+(S1-7), (A1-74)+(S1-11), (A1-74)+(S2-1), (A1-74)+(S3-1), (A1-74)+(S3-2), (A1-74)+(S3-4), (A1-74)+(S3-7), (A1-74)+(S3-8), (A1-74)+(S3-10), (A1-74)+(S3-11), (A1-74)+(S4-1), (A1-74)+(S4-5), (A1-74)+(S4-6), (A1-74)+(S11-1), (A1-74)+(S11-2), (A1-74)+(S11-3), (A1-74)+(S13-1), (A1-74)+(S13-2), (A1-74)+(S13-3), (A1-74)+(S13-8), (A1-74)+(S14-1), (A1-74)+(S14-2), (A1-74)+(S14-3),
(A1-75)+(S1-1), (A1-75)+(S1-7), (A1-75)+(S1-11), (A1-75)+(S2-1), (A1-75)+(S3-1), (A1-75)+(S3-2), (A1-75)+(S3-4), (A1-75)+(S3-7), (A1-75)+(S3-8), (A1-75)+(S3-10), (A1-75)+(S3-11), (A1-75)+(S4-1), (A1-75)+(S4-5), (A1-75)+(S4-6), (A1-75)+(S11-1), (A1-75)+(S11-2), (A1-75)+(S11-3), (A1-75)+(S13-1), (A1-75)+(S13-2), (A1-75)+(S13-3), (A1-75)+(S13-8), (A1-75)+(S14-1), (A1-75)+(S14-2), (A1-75)+(S14-3),
(A1-76)+(S1-1), (A1-76)+(S1-7), (A1-76)+(S1-11), (A1-76)+(S2-1), (A1-76)+(S3-1), (A1-76)+(S3-2), (A1-76)+(S3-4), (A1-76)+(S3-7), (A1-76)+(S3-8), (A1-76)+(S3-10), (A1-76)+(S3-11), (A1-76)+(S4-1), (A1-76)+(S4-5), (A1-76)+(S4-6), (A1-76)+(S11-1), (A1-76)+(S11-2), (A1-76)+(S11-3), (A1-76)+(S13-1), (A1-76)+(S13-2), (A1-76)+(S13-3), (A1-76)+(S13-8), (A1-76)+(S14-1), (A1-76)+(S14-2), (A1-76)+(S14-3),
(A1-77)+(S1-1), (A1-77)+(S1-7), (A1-77)+(S1-11), (A1-77)+(S2-1), (A1-77)+(S3-1), (A1-77)+(S3-2), (A1-77)+(S3-4), (A1-77)+(S3-7), (A1-77)+(S3-8), (A1-77)+(S3-10), (A1-77)+(S3-11), (A1-77)+(S4-1), (A1-77)+(S4-5), (A1-77)+(S4-6), (A1-77)+(S11-1), (A1-77)+(S11-2), (A1-77)+(S11-3), (A1-77)+(S13-1), (A1-77)+(S13-2), (A1-77)+(S13-3), (A1-77)+(S13-8), (A1-77)+(S14-1), (A1-77)+(S14-2), (A1-77)+(S14-3),
(A2-1)+(S1-1), (A2-1)+(S1-7), (A2-1)+(S1-11), (A2-1)+(S2-1), (A2-1)+(S3-1), (A2-1)+(S3-2), (A2-1)+(S3-4), (A2-1)+(S3-7), (A2-1)+(S3-8), (A-1)+(S3-10), (A2-1)+(S3-11), (A2-1)+(S4-1), (A2-1)+(S4-5), (A2-1)+(S4-6), (A2-1)+(S11-1), (A2-1)+(S11-2), (A2-1)+(S11-3), (A2-1)+(S13-1), (A2-1)+(S13-2), (A2-1)+(S13-3), (A2-1)+(S13-8), (A2-1)+(S14-1), (A2-1)+(S14-2), (A2-1)+(S14-3),
(A2-2)+(S1-1), (A2-2)+(S1-7), (A2-2)+(S1-11), (A2-2)+(S2-1), (A2-2)+(S3-1), (A2-2)+(S3-2), (A2-2)+(S3-4), (A2-2)+(S3-7), (A2-2)+(S3-8), (A2-2)+(S3-10), (A2-2)+(S3-11), (A2-2)+(S4-1), (A2-2)+(S4-5), (A2-2)+(S4-6), (A2-2)+(S11-1), (A2-2)+(S11-2), (A2-2)+(S11-3), (A2-2)+(S13-1), (A2-2)+(S13-2), (A2-2)+(S13-3), (A2-2)+(S13-8), (A2-2)+(S14-1), (A2-2)+(S14-2), (A2-2)+(S14-3),
(A2-3)+(S1-1), (A2-3)+(S1-7), (A2-3)+(S1-11), (A2-3)+(S2-1), (A2-3)+(S3-1), (A2-3)+(S3-2), (A2-3)+(S3-4), (A2-3)+(S3-7), (A2-3)+(S3-8), (A2-3)+(S3-10), (A2-3)+(S3-11), (A2-3)+(S4-1), (A2-3)+(54-5), (A2-3)+(S4-6), (A2-3)+(S11-1), (A2-3)+(S11-2), (A2-3)+(S11-3), (A2-3)+(S13-1), (A2-3)+(S13-2), (A2-3)+(S13-3), (A2-3)+(S13-8), (A2-3)+(S14-1), (A2-3)+(S14-2), (A2-3)+(S14-3),
(A2-4)+(S1-1), (A2-4)+(S1-7), (A2-4)+(S1-11), (A2-4)+(S2-1), (A2-4)+(S3-1), (A2-4)+(S3-2), (A2-4)+(S3-4), (A2-4)+(S3-7), (A2-4)+(S3-8), (A2-4)+(S3-10), (A2-4)+(S3-11), (A2-4)+(S4-1), (A2-4)+(S4-5), (A2-4)+(S4-6), (A2-4)+(S11-1), (A2-4)+(S11-2), (A2-4)+(S11-3), (A2-4)+(S13-1), (A2-4)+(S13-2), (A2-4)+(S13-3), (A2-4)+(S13-8), (A2-4)+(S14-1), (A2-4)+(S14-2), (A2-4)+(S14-3),
(A2-5)+(S1-1), (A2-5)+(S1-7), (A2-5)+(S1-11), (A2-5)+(S2-1), (A2-5)+(S3-1), (A2-5)+(S3-2), (A2-5)+(S3-4), (A2-5)+(S3-7), (A2-5)+(S3-8), (A2-5)+(S3-10), (A2-5)+(S3-11), (A2-5)+(S4-1), (A2-5)+(S4-5), (A2-5)+(S4-6), (A2-5)+(S11-1), (A2-5)+(S11-2), (A2-5)+(S11-3), (A2-5)+(S13-1), (A2-5)+(S13-2), (A2-5)+(S13-3), (A2-5)+(S13-8), (A2-5)+(S14-1), (A2-5)+(S14-2), (A2-5)+(S14-3),
(A2-6)+(S1-1), (A2-6)+(S1-7), (A2-6)+(S1-11), (A2-6)+(S2-1), (A2-6)+(S3-1), (A2-6)+(S3-2), (A2-6)+(S3-4), (A2-6)+(S3-7), (A2-6)+(S3-8), (A2-6)+(S3-10), (A2-6)+(S3-11), (A2-6)+(S4-1), (A2-6)+(S4-5), (A2-6)+(S4-6), (A2-6)+(S11-1), (A2-6)+(S11-2), (A2-6)+(S11-3), (A2-6)+(S13-1), (A2-6)+(S13-2), (A2-6)+(S13-3), (A2-6)+(S13-8), (A2-6)+(S14-1), (A2-6)+(S14-2), (A2-6)+(S14-3),
(A2-7)+(S1-1), (A2-7)+(S1-7), (A2-7)+(S1-11), (A2-7)+(S2-1), (A2-7)+(S3-1), (A2-7)+(S3-2), (A2-7)+(S3-4), (A2-7)+(S3-7), (A2-7)+(S3-8), (A2-7)+(S3-10), (A2-7)+(S3-11), (A2-7)+(S4-1), (A2-7)+(S4-5), (A2-7)+(S4-6), (A2-7)+(S11-1), (A2-7)+(S11-2), (A2-7)+(S11-3), (A2-7)+(S13-1), (A2-7)+(S13-2), (A2-7)+(S13-3), (A2-7)+(S13-8), (A2-7)+(S14-1), (A2-7)+(S14-2), (A2-7)+(S14-3),
(A2-8)+(S1-1), (A2-8)+(S1-7), (A2-8)+(S1-11), (A2-8)+(S2-1), (A2-8)+(S3-1), (A2-8)+(S3-2), (A2-8)+(S3-4), (A2-8)+(S3-7), (A2-8)+(S3-8), (A2-8)+(S3-10), (A2-8)+(S3-11), (A2-8)+(S4-1), (A2-8)+(S4-5), (A2-8)+(S4-6), (A2-8)+(S11-1), (A2-8)+(S11-2), (A2-8)+(S11-3), (A2-8)+(S13-1), (A2-8)+(S13-2), (A2-8)+(S13-3), (A2-8)+(S13-8), (A2-8)+(S14-1), (A2-8)+(S14-2), (A2-8)+(S14-3),
(A3-1)+(S1-1), (A3-1)+(S1-7), (A3-1)+(S1-11), (A3-1)+(S2-1), (A3-1)+(S3-1), (A3-1)+(S3-2), (A3-1)+(S3-4), (A3-1)+(S3-7), (A3-1)+(S3-8), (A3-1)+(S3-10), (A3-1)+(S3-11), (A3-1)+(S4-1), (A3-1)+(S4-5), (A3-1)+(S4-6), (A3-1)+(S11-1), (A3-1)+(S11-2), (A3-1)+(S11-3), (A3-1)+(S13-1), (A3-1)+(S13-2), (A3-1)+(S13-3), (A3-1)+(S13-8), (A3-1)+(S14-1), (A3-1)+(S14-2), (A3-1)+(S14-3),
(A3-2)+(S1-1), (A3-2)+(S1-7), (A3-2)+(S1-11), (A3-2)+(S2-1), (A3-2)+(S3-1), (A3-2)+(S3-2), (A3-2)+(S3-4), (A3-2)+(S3-7), (A3-2)+(S3-8), (A3-2)+(S3-10), (A3-2)+(S3-11), (A3-2)+(S4-1), (A3-2)+(S4-5), (A3-2)+(S4-6), (A3-2)+(S11-1), (A3-2)+(S11-2), (A3-2)+(S11-3), (A3-2)+(S13-1), (A3-2)+(S13-2), (A3-2)+(S13-3), (A3-2)+(S13-8), (A3-2)+(S14-1), (A3-2)+(S14-2), (A3-2)+(S14-3),
(A3-3)+(S1-1), (A3-3)+(S1-7), (A3-3)+(S1-11), (A3-3)+(S2-1), (A3-3)+(S3-1), (A3-3)+(S3-2), (A3-3)+(S3-4), (A3-3)+(S3-7), (A3-3)+(S3-8), (A3-3)+(S3-10), (A3-3)+(S3-11), (A3-3)+(S4-1), (A3-3)+(S4-5), (A3-3)+(S4-6), (A3-3)+(S11-1), (A3-3)+(S11-2), (A3-3)+(S11-3), (A3-3)+(S13-1), (A3-3)+(S13-2), (A3-3)+(S13-3), (A3-3)+(S13-8), (A3-3)+(S14-1), (A3-3)+(S14-2), (A3-3)+(S14-3),
(A3-4)+(S1-1), (A3-4)+(S1-7), (A3-4)+(S1-11), (A3-4)+(S2-1), (A3-4)+(S3-1), (A3-4)+(S3-2), (A3-4)+(S3-4), (A3-4)+(S3-7), (A3-4)+(S3-8), (A3-4)+(S3-10), (A3-4)+(S3-11), (A3-4)+(S4-1), (A3-4)+(S4-5), (A3-4)+(S4-6), (A3-4)+(S11-1), (A3-4)+(S11-2), (A3-4)+(S11-3), (A3-4)+(S13-1), (A3-4)+(S13-2), (A3-4)+(S13-3), (A3-4)+(S13-8), (A3-4)+(S14-1), (A3-4)+(S14-2), (A3-4)+(S14-3),
(A3-5)+(S1-1), (A3-5)+(S1-7), (A3-5)+(S1-11), (A3-5)+(S2-1), (A3-5)+(S3-1), (A3-5)+(S3-2), (A3-5)+(S3-4), (A3-5)+(S3-7), (A3-5)+(S3-8), (A3-5)+(S3-10), (A3-5)+(S3-11), (A3-5)+(S4-1), (A3-5)+(S4-5), (A3-5)+(S4-6), (A3-5)+(S11-1), (A3-5)+(S11-2), (A3-5)+(S11-3), (A3-5)+(S13-1), (A3-5)+(S13-2), (A3-5)+(S13-3), (A3-5)+(S13-8), (A3-5)+(S14-1), (A3-5)+(S14-2), (A3-5)+(S14-3),
(A3-6)+(S1-1), (A3-6)+(S1-7), (A3-6)+(S1-11), (A3-6)+(S2-1), (A3-6)+(S3-1), (A3-6)+(S3-2), (A3-6)+(S3-4), (A3-6)+(S3-7), (A3-6)+(S3-8), (A3-6)+(S3-10), (A3-6)+(S3-11), (A3-6)+(S4-1), (A3-6)+(S4-5), (A3-6)+(S4-6), (A3-6)+(S11-1), (A3-6)+(S11-2), (A3-6)+(S11-3), (A3-6)+(S13-1), (A3-6)+(S13-2), (A3-6)+(S13-3), (A3-6)+(S13-8), (A3-6)+(S14-1), (A3-6)+(S14-2), (A3-6)+(S14-3),
(A3-7)+(S1-1), (A3-7)+(S1-7), (A3-7)+(S1-11), (A3-7)+(S2-1), (A3-7)+(S3-1), (A3-7)+(S3-2), (A3-7)+(S3-4), (A3-7)+(S3-7), (A3-7)+(S3-8), (A3-7)+(S3-10), (A3-7)+(S3-11), (A3-7)+(S4-1), (A3-7)+(S4-5), (A3-7)+(S4-6), (A3-7)+(S11-1), (A3-7)+(S11-2), (A3-7)+(S11-3), (A3-7)+(S13-1), (A3-7)+(S13-2), (A3-7)+(S13-3), (A3-7)+(S13-8), (A3-7)+(S14-1), (A3-7)+(S14-2), (A3-7)+(S14-3),
(A4-1)+(S1-1), (A4-1)+(S1-7), (A4-1)+(S1-11), (A4-1)+(S2-1), (A4-1)+(S3-1), (A4-1)+(S3-2), (A4-1)+(S3-4), (A4-1)+(S3-7), (A4-1)+(S3-8), (A4-1)+(S3-10), (A4-1)+(S3-11), (A4-1)+(S4-1), (A4-1)+(S4-5), (A4-1)+(S4-6), (A4-1)+(S11-1), (A4-1)+(S11-2), (A4-1)+(S11-3), (A4-1)+(S13-1), (A4-1)+(S13-2), (A4-1)+(S13-3), (A4-1)+(S13-8), (A4-1)+(S14-1), (A4-1)+(S14-2), (A4-1)+(S14-3),
(A4-2)+(S1-1), (A4-2)+(S1-7), (A4-2)+(S1-11), (A4-2)+(S2-1), (A4-2)+(S3-1), (A4-2)+(S3-2), (A4-2)+(S3-4), (A4-2)+(S3-7), (A4-2)+(S3-8), (A4-2)+(S3-10), (A4-2)+(S3-11), (A4-2)+(S4-1), (A4-2)+(S4-5), (A4-2)+(S4-6), (A4-2)+(S11-1), (A4-2)+(S11-2), (A4-2)+(S11-3), (A4-2)+(S13-1), (A4-2)+(S13-2), (A4-2)+(S13-3), (A4-2)+(S13-8), (A4-2)+(S14-1), (A4-2)+(S14-2), (A4-2)+(S14-3),
(A4-3)+(S1-1), (A4-3)+(S1-7), (A4-3)+(S1-11), (A4-3)+(S2-1), (A4-3)+(S3-1), (A4-3)+(S3-2), (A4-3)+(S3-4), (A4-3)+(S3-7), (A4-3)+(S3-8), (A4-3)+(S3-10), (A4-3)+(S3-11), (A4-3)+(S4-1), (A4-3)+(S4-5), (A4-3)+(S4-6), (A4-3)+(S11-1), (A4-3)+(S11-2), (A4-3)+(S11-3), (A4-3)+(S13-1), (A4-3)+(S13-2), (A4-3)+(S13-3), (A4-3)+(S13-8), (A4-3)+(S14-1), (A4-3)+(S14-2), (A4-3)+(S14-3),
(A4-4)+(S1-1), (A4-4)+(S1-7), (A4-4)+(S1-11), (A4-4)+(S2-1), (A4-4)+(S3-1), (A4-4)+(S3-2), (A4-4)+(S3-4), (A4-4)+(S3-7), (A4-4)+(S3-8), (A4-4)+(S3-10), (A4-4)+(S3-11), (A4-4)+(S4-1), (A4-4)+(S4-5), (A4-4)+(S4-6), (A4-4)+(S11-1), (A4-4)+(S11-2), (A4-4)+(S11-3), (A4-4)+(S13-1), (A4-4)+(S13-2), (A4-4)+(S13-3), (A4-4)+(S13-8), (A4-4)+(S14-1), (A4-4)+(S14-2), (A4-4)+(S14-3),
(A4-5)+(S1-1), (A4-5)+(S1-7), (A4-5)+(S1-11), (A4-5)+(S2-1), (A4-5)+(S3-1), (A4-5)+(S3-2), (A4-5)+(S3-4), (A4-5)+(S3-7), (A4-5)+(S3-8), (A4-5)+(S3-10), (A4-5)+(S3-11), (A4-5)+(S4-1), (A4-5)+(S4-5), (A4-5)+(S4-6), (A4-5)+(S11-1), (A4-5)+(S11-2), (A4-5)+(S11-3), (A4-5)+(S13-1), (A4-5)+(S13-2), (A4-5)+(S13-3), (A4-5)+(S13-8), (A4-5)+(S14-1), (A4-5)+(S14-2), (A4-5)+(S14-3),
(A4-6)+(S1-1), (A4-6)+(S1-7), (A4-6)+(S1-11), (A4-6)+(S2-1), (A4-6)+(S3-1), (A4-6)+(S3-2), (A4-6)+(S3-4), (A4-6)+(S3-7), (A4-6)+(S3-8), (A4-6)+(S3-10), (A4-6)+(S3-11), (A4-6)+(S4-1), (A4-6)+(S4-5), (A4-6)+(S4-6), (A4-6)+(S11-1), (A4-6)+(S11-2), (A4-6)+(S11-3), (A4-6)+(S13-1), (A4-6)+(S13-2), (A4-6)+(S13-3), (A4-6)+(S13-8), (A4-6)+(S14-1), (A4-6)+(S14-2), (A4-6)+(S14-3),
(A4-7)+(S1-1), (A4-7)+(S1-7), (A4-7)+(S1-11), (A4-7)+(S2-1), (A4-7)+(S3-1), (A4-7)+(S3-2), (A4-7)+(S3-4), (A4-7)+(S3-7), (A4-7)+(S3-8), (A4-7)+(S3-10), (A4-7)+(S3-11), (A4-7)+(S4-1), (A4-7)+(S4-5), (A4-7)+(S4-6), (A4-7)+(S11-1), (A4-7)+(S11-2), (A4-7)+(S11-3), (A4-7)+(S13-1), (A4-7)+(S13-2), (A4-7)+(S13-3), (A4-7)+(S13-8), (A4-7)+(S14-1), (A4-7)+(S14-2), (A4-7)+(S14-3),
(A4-8)+(S1-1), (A4-8)+(S1-7), (A4-8)+(S1-11), (A4-8)+(S2-1), (A4-8)+(S3-1), (A4-8)+(S3-2), (A4-8)+(S3-4), (A4-8)+(S3-7), (A4-8)+(S3-8), (A4-8)+(S3-10), (A4-8)+(S3-11), (A4-8)+(S4-1), (A4-8)+(S4-5), (A4-8)+(S4-6), (A4-8)+(S11-1), (A4-8)+(S11-2), (A4-8)+(S11-3), (A4-8)+(S13-1), (A4-8)+(S13-2), (A4-8)+(S13-3), (A4-8)+(S13-8), (A4-8)+(S14-1), (A4-8)+(S14-2), (A4-8)+(S14-3),
(A4-9)+(S1-1), (A4-9)+(S1-7), (A4-9)+(S1-11), (A4-9)+(S2-1), (A4-9)+(S3-1), (A4-9)+(S3-2), (A4-9)+(S3-4), (A4-9)+(S3-7), (A4-9)+(S3-8), (A4-9)+(S3-10), (A4-9)+(S3-11), (A4-9)+(S4-1), (A4-9)+(S4-5), (A4-9)+(S4-6), (A4-9)+(S11-1), (A4-9)+(S11-2), (A4-9)+(S11-3), (A4-9)+(S13-1), (A4-9)+(S13-2), (A4-9)+(S13-3), (A4-9)+(S13-8), (A4-9)+(S14-1), (A4-9)+(S14-2), (A4-9)+(S14-3).

Im Rahmen der vorliegenden Erfindung sind insbesondere Zusammensetzungen der Herbizide (A) mit folgenden Safenern bevorzugt: Mefenpyr-diethyl, Fenchlorazol, Isoxadifen-ethyl, Cloquintocet-mexyl, Dichlormid, 3-Dichloracetyl-2,2,5-trimethyl-1,3-oxazolidin, Benoxacor, 3-Dichloracetyl-1-oxa-3-aza-spiro[4,5]decan, 1-Dichloracetyl-azepan, Furilazol, ((R)-3-Dichloracetyl-5-(2-furyl)-2,2-dimethyloxazolidin), Cyprosulfamid, N-Isopropyl-4-sulfamoylbenzamid-1-(2-methoxyphenyl)ethanon, 1-[4-(N-2-Methoxybenzoylsulfamoyl)phenyl]-3-methylharnstoff, Oxabetrinil, Fluxofenim, Cyometrinil, 1,8-Naphthalindicarbonsäure-anhydrid, Fenclorim, Flurazole, Dietholate, Dimepiperate, Daimuron und Cumyluron.

Im Rahmen der vorliegenden Erfindung sind ganz besonders bevorzugt Zusammensetzungen der Herbizide (A) mit den Safenern: Daimuron, Benoxacor, Furilazol, Fluxofenim, Fenchlorazol(-ethylester), Mefenpyr-diethyl, Cloquintocet-mexyl, Isoxadifen-ethyl, Cyprosulfamide, Flurazole, Oxabetrinil, Dichlormid und Dietholate.

Im Rahmen der vorliegenden Erfindung sind noch bevorzugter Zusammensetzungen der Herbizide (A) mit den folgenden Safenern: Mefenpyr-diethyl, Isoxadifen-ethyl, Cyprosulfamide, Fenchlorazol-ethylester, Benoxacor, Cloquintocet-mexyl, Fluxofenim und Furilazol.

Bevorzugt sind Herbizid-Safener-Zusammensetzungen, enthaltend (A) eine herbizid wirksame Menge an einer oder mehrerer Verbindungen der Formel (I) oder deren Salzen, und (B) eine antidotisch-wirksame Menge an einem oder mehreren Safenern.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenern, die geeignet ist, die phytotoxische Wirkung von Pflanzenschutzmittelwirkstoffen (z.B. von Herbiziden) an Kulturpflanzen zu reduzieren.

Die Safener (B) eignen sich zur Reduktion phytotoxischer Effekte, die beim Einsatz von Herbiziden der allgemeinen Formel (I) in Nutzpflanzenkulturen auftreten können, ohne die Wirksamkeit dieser herbiziden Wirkstoffe gegen Schadpflanzen wesentlich zu beeinträchtigen. Hierdurch kann das Einsatzgebiet herkömmlicher Pflanzenschutzmittel ganz erheblich erweitert z.B. auf Kulturen, in denen bisher ein Einsatz der Herbizide nicht möglich oder nur beschränkt möglich war.

Die benötigten Aufwandmengen der Safener können je nach Indikation und verwendetem herbiziden Wirkstoff innerhalb weiter Grenzen schwanken und sind in der Regel im Bereich von 0,001 bis 5 kg, vorzugsweise 0,005 bis 2,5 kg und besonders 0,05 bis 0,5 kg Wirkstoff je Hektar.

Die Herbizide (A) und die Safener (B) können zusammen (z.B. als fertige Formulierung oder im Tank-mix-Verfahren) oder in beliebiger Reihenfolge nacheinander ausgebracht werden, z.B. durch Sprüh-, Gieß- und Spritzanwendung oder durch Granulatstreuung. Das Gewichtsverhältnis Herbizid der allgemeinen Formel (I) (A) : Safener (B) kann innerhalb weiter Grenzen variieren und liegt vorzugsweise im Bereich von 1:10000 bis 10000:1, insbesondere von 1:1000 bis 1000:1 1 und ganu besonders von 1:20 bis 20:1. Die jeweils optimalen Mengen an der allgemeinen Formel (I) (A) und Safener (B) sind vom Typ des verwendeten Herbizids und des verwendeten Safeners sowie von der Art und dem Entwicklungsstadium des zu behandelnden Pflanzenbestandes abhängig und lassen sich von Fall zu Fall durch einfache, routinemäßige Vorversuche ermitteln.

Die in den erfindungsgemäßen Herbizid-Safener-Zusammensetzungen enthaltenen Safener (B) können je nach ihren Eigenschaften zur Vorbehandlung des Saatgutes der Kulturpflanze (z.B. zur Beizung des Saatguts) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche (einschließlich eventuell auf der Anbaufläche befindlichen Wassers, z.B. bei Reisapplikationen) vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

In einer bevorzugten Ausführungsform werden das Saatgut (z.B. Körner, Samen oder vegetative Vermehrungsorgane wie Knollen oder Sprossteile mit Knospen) oder Setzlinge mit den Safenern (B), gegebenenfalls in Kombination mit anderen agrochemischen Wirkstoffen, vorbehandelt. Zur Vorbehandlung des Saatguts können die Wirkstoffe z.B. durch Beizung an das Saatgut gebracht oder die Wirkstoffe und das Saatgut können in Wasser oder andere Lösungsmittel gegeben, und die Wirkstoffe z.B. durch Anlagerung oder Diffusion im Tauchverfahren oder durch Quellen oder Vorkeimen aufgenommen werden. Zur Vorbehandlung von Setzlingen können die jungen Pflanzen z.B. durch Spritzen, Tauchen oder Gießen mit den Safenern, gegebenenfalls in Kombination mit anderen agrochemischen Wirkstoffen, in Kontakt gebracht und anschließend verpflanzt und gegebenenfalls mit den Herbiziden (A) nachbehandelt werden.

Die Saatgut- oder Setzlingsbehandlung kann mit den Safenern (B) alleine oder gemeinsam mit anderen agrochemischen Wirkstoffen - wie Fungiziden, Insektiziden oder Mitteln zur Pflanzenstärkung, Düngung oder zur Beschleunigung der Quell- und Keimungsvorgänge - erfolgen. Dabei können die Safener nach der Vorbehandlungsanwendung anschließend nochmals vor, nach oder gemeinsam mit einem oder mehreren Herbiziden (A) eventuell auch in Kombination mit anderen bekannten Herbiziden angewandt werden. Durch die Vorbehandlung des Saatguts oder der Setzlinge kann eine verbesserte Langzeitwirkung der Safener erzielt werden.

Gegenstand der vorliegenden Erfindung ist somit weiterhin ein Verfahren zur Bekämpfung von unerwünschten Pflanzen in Pflanzenkulturen, das dadurch gekennzeichnet ist, dass die Herbizide (A) und die Safener (B) der erfindungsgemäßen Herbizid-Safener-Zusammensetzungen auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden, z.B. gemeinsam oder getrennt. Dabei können einer oder mehrere Safener (B), vorzugsweise eine oder mehrere, insbesondere eine, Verbindung der Gruppen (S1) bis (S15) vor, nach oder gleichzeitig mit dem oder den Herbizid(en) der allgemeinen Formel (I) (A) auf die Pflanzen, das Saatgut oder die Fläche, auf der die Pflanzen wachsen (z.B. die Anbaufläche), appliziert werden. In einer bevorzugten Ausführungsform werden die Safener (B) zur Saatgutbehandlung eingesetzt.

Unter unerwünschten Pflanzen sind alle Pflanzen zu verstehen, die an Orten wachsen, wo sie unerwünscht sind. Dies können z.B. Schadpflanzen (z.B. mono- oder dikotyle Unkräuter oder unerwünschte Kulturpflanzen) sein, z.B. auch solche, die gegen bestimmte herbizide Wirkstoffe wie Glyphosate, Atrazin, Glufosinate oder Imidazolinon-Herbizide resistent sind.

Monokotyle Unkräuter entstammen z.B. den Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera. Dikotyle Unkräuter entstammen z.B. den Gattungen Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum, Euphorbia.

Bevorzugt wird in dem erfindungsgemäßen Verfahren eine wirksame Menge der Herbizide (A) und der Safener (B) zur Bekämpfung von Schadpflanzen angewendet in Pflanzenkulturen, beispielsweise in wirtschaftlich bedeutenden Ackerbaukulturen z.B. monokotylen Ackerbaukulturen wie Getreide (z.B. Weizen, Gerste, Roggen, Hafer), Reis, Mais, Hirse, oder dikotylen Ackerbaukulturen wie Zuckerrübe, Raps, Baumwolle, Sonnenblumen und Leguminosen z.B. der Gattungen Glycine (z.B. Glycine max. wie nicht-transgene Glycine max. (z.B. konventionelle Sorten wie STS-Sorten) oder transgene Glycine max. (z.B. RR-Soja oder LL-Soja) und deren Kreuzungen), Phaseolus, Pisum, Vicia und Arachis, oder Gemüsekulturen aus verschiedenen botanischen Gruppen wie Kartoffel, Lauch, Kohl, Karotte, Tomate, Zwiebel, sowie Dauer- und Plantagenkulturen wie Kern- und Steinobst, Beerenobst, Wein, Hevea, Bananen, Zuckerrohr, Kaffee, Tee, Citrus, Nussplantagen, Rasen, Palmenkulturen und Forstkulturen.

Gegenstand der Erfindung ist auch die Verwendung der erfindungsgemäßen Herbizid-Safener-Zusammensetzungen zur Bekämpfung von unerwünschtem Pflanzenwuchs, vorzugsweise in Pflanzenkulturen.

Die erfindungsgemäßen Herbizid-Safener-Zusammensetzungen können nach bekannten Verfahren z.B. als Mischformulierungen der Einzelkomponenten, gegebenenfalls mit weiteren Wirkstoffen, Zusatzstoffen und/oder üblichen Formulierungshilfsmitteln hergestellt werden, die dann in üblicher Weise mit Wasser verdünnt zur Anwendung gebracht werden, oder als sogenannte Tankmischungen durch gemeinsame Verdünnung der getrennt formulierten oder partiell getrennt formulierten Einzelkomponenten mit Wasser hergestellt werden. Ebenfalls möglich ist die zeitlich versetzte Anwendung (Splitapplikation) der getrennt formulierten oder partiell getrennt formulierten Einzelkomponenten. Möglich ist auch die Anwendung der Einzelkomponenten oder der Herbizid-Safener-Zusammensetzungen in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Zusammensetzung.

Die erfindungsgemäßen Herbizid-Safener-Zusammensetzungen können auch zur Bekämpfung von Schadpflanzen in Kulturen von bekannten oder noch zu entwickelnden gentechnisch veränderten Pflanzen eingesetzt werden.

Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Herbizid-Safener-Zusammensetzungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Herbizide (A) in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624).

Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen erfindungsgemäßen Herbizid-Safener-Zusammensetzungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen erfindungsgemäßen Herbizid-Safener-Zusammensetzungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen erfindungsgemäßen Herbizid-Safener-Zusammensetzungen zur Bekämpfung von Schadpflanzen in transgenen Kulturpflanzen.

Bevorzugt ist die Anwendung der erfindungsgemäßen Zusammensetzungen in wirtschaftlich bedeutenden transgenen Kulturen von Nutz- und Zierpflanzen, z. B. von Getreide (z.B. Weizen, Gerste, Roggen, Hafer), Hirse, Reis, Maniok und Mais oder auch Kulturen von Zuckerrübe, Baumwolle, Soja, Raps, Kartoffel, Tomate, Erbse und anderen Gemüsekulturen.

Gegenstand der Erfindung ist deshalb auch die Verwendung der erfindungsgemäßen Herbizid-Safener-Zusammensetzungen zur Bekämpfung von Schadpflanzen in transgenen Kulturpflanzen oder Kulturpflanzen, die Toleranz durch Selektionszüchtung aufweisen.

Die Herbizide (A) und die Safener (B) können gemeinsam oder getrennt in übliche Formulierungen z.B. zur Sprüh-, Gieß-, Spritz- und Saatgutbeizanwendung übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen. Die Formulierungen können die üblichen Hilfs- und Zusatzstoffe enthalten.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 Gew.-%.

Die Herbizide (A) und die Safener (B) können als solche oder in ihren Formulierungen auch in Mischung mit anderen agrochemischen Wirkstoffen, wie bekannten Herbiziden, zur Bekämpfung von unerwünschtem Pflanzenwuchs, z.B. zur Unkrautbekämpfung oder zur Bekämpfung von unerwünschten Kulturpflanzen Verwendung finden, wobei z.B. Fertigformulierungen oder Tankmischungen möglich sind.

Auch Mischungen mit anderen bekannten Wirkstoffen wie Fungiziden, Insektiziden, Akariziden, Nematiziden, Schutzstoffen gegen Vogelfraß, Pflanzennährstoffen und Bodenstrukturverbesserungsmitteln sind möglich, ebenso mit im Pflanzenschutz üblichen Zusatzstoffen und Formulierungshilfsmitteln.

Die Herbizide (A) und die Safener (B) können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht üblicherweise, z.B. durch Gießen, Spritzen, Sprühen, Streuen.

Die Wirkstoffe können auf die Pflanzen, Pflanzenteile, das Saatgut oder die Anbaufläche (Ackerboden) ausgebracht werden, vorzugsweise auf das Saatgut oder die grünen Pflanzen und Pflanzenteile und gegebenenfalls zusätzlich auf den Ackerboden. Eine Möglichkeit der Anwendung ist die gemeinsame Ausbringung der Wirkstoffe in Form von Tankmischungen, wobei die optimal formulierten konzentrierten Formulierungen der Einzelwirkstoffe gemeinsam im Tank mit Wasser gemischt und die erhaltene Spritzbrühe ausgebracht wird.

Eine gemeinsame Formulierung der erfindungsgemäßen Zusammensetzungen an Herbizid (A) und Safener (B) hat den Vorteil der leichteren Anwendbarkeit, weil die Mengen der Komponenten bereits im optimalen Verhältnis zueinander eingestellt werden können. Außerdem können die Hilfsmittel in der Formulierung aufeinander optimal abgestimmt werden.

Als Kombinationspartner für die erfindungsgemäße Herbizid-Safener-Zusammensetzungen in Mischungsformulierungen oder im Tank-Mix sind beispielsweise bekannte, vorzugsweise herbizide Wirkstoffe, die auf einer Inhibition von beispielsweise Acetolactat-Synthase, Acetyl-Coenzym-A-Carboxylase, PS 1, PS II, HPPD, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder in dem Handbuch "The Pesticide Manual", 12. Auflage 2000, oder 13. Auflage 2003 oder 14. Auflage 2006/2007, oder in dem entsprechenden "e-Pesticide Manual", Version 5.0 (2008-10), jeweils herausgegeben vom British Crop Protection Council, (im Folgenden auch kurz "PM"), und dort zitierter Literatur beschrieben. Listen von "Common names" sind auch in "The Compendium of Pesticide Common Names" im Internet verfügbar. Als literaturbekannte Herbizide, die mit den erfindungsgemäßen Herbizid-Safener-Zusammensetzungen kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet und umfassen stets sämtliche Anwendungsformen wie Säuren, Salze, Ester und Isomere wie Stereoisomere und optische Isomere. Dabei sind eine und zum Teil auch mehrere Anwendungsformen genannt):

2,4-D, Acetochlor, Acifluorfen, Acifluorfen-sodium, Aclonifen, Alachlor, Alloxydim, Alloxydim-sodium, Ametryn, Amicarbazone, Amidosulfuron, Aminopyralid, Amitrole, Anilofos, Asulam, Atrazine, Azafenidin, Azimsulfuron, Beflubutamid, Benazolin, Benazolin-ethyl, Benfuresate, Bensulfuron-methyl, Bentazone, Benzfendizone, Benzobicyclon, Benzofenap, Bifenox, Bilanafos, Bispyribac-natrium, Bromacil, Bromobutide, Bromofenoxim, Bromoxynil, Butachlor, Butafenacil, Butenachlor, Butralin, Butroxydim, Butylate, Cafenstrole, Carbetamide, Carfentrazone-ethyl, Chlomethoxyfen, Chloridazon, Chlorimuron-ethyl, Chlornitrofen, Chlortoluron, Chlorsulfuron, Cinidon-ethyl, Cinmethylin, Cinosulfuron, Clefoxydim, Clethodim, Clodinafop-propargyl, Clomazone, Clomeprop, Clopyralid, Cloransulam-methyl, Cumyluron, Cyanazine, Cyclosulfamuron, Cycloxydim, Cyhalofop-butyl, Desmedipham, Dicamba, Dichlobenil, Dichlorprop, Dichlorprop-P, Diclofop-methyl, Diclosulam, Difenzoquat, Diflufenican, Diflufenzopyr, Dikegulac-sodium, Dimefuron, Dimepiperate, Dimethachlor, Dimethametryn, Dimethenamid, Triaziflam, Diquatdibromide, Dithiopyr, Diuron, Dymron, EPTC, Esprocarb, Ethalfluralin, Ethametsulfuron-methyl, Ethofumesate, Ethoxyfen, Ethoxysulfuron, Etobenzanid, Fenoxaprop-ethyl, Fenoxaprop-P-ethyl, Fentrazamide, Flamprop-M-isopropyl, Flamprop-M-methyl, Flazasulfuron, Florasulam, Fluazifop, Fluazifop-butyl, Fluazifop-P-butyl, Fluazolate, Flucarbazone-sodium, Flucetosulfuron, Fluchloralin, Flufenacet,

Flufenpyr, Flumetsulam, Flumiclorac-pentyl, Flumioxazin, Fluometuron, Fluorchloridone, Fluorglycofen-ethyl, Flupoxam, Flupyrsulfuron-methyl-sodium, Fluridone, Fluroxypyr, Fluroxypyr-butoxypropyl, Fluroxypyr-meptyl, Flurprimidol, Flurtamone, Fluthiacet-methyl, Fomesafen, Foramsulfuron, Glufosinate, Glufosinate-P, Glufosinate-ammonium, Glufosinate-P-ammonium, Glufosinate-P-sodium, Glyphosate, Halosulfuron-methyl, Haloxyfop, Haloxyfop-ethoxyethyl, Haloxyfop-methyl, Haloxyfop-P-methyl, Hexazinone, Imazamethabenz-methyl, Imazamox, Imazapic, Imazapyr, Imazaquin, Imazethapyr, Imazosulfuron, Indanofan, Indaziflam, Iodosulfuron-methylnatrium, Ioxynil, Isoproturon, Isouron, Isoxaben, Isoxachlortole, Isoxaflutole, Ketospiradox, Lactofen, Lenacil, Linuron, MCPA, Mecoprop, Mecoprop-P, Mefenacet, Mesosulfuron-methyl, Mesotrione, Metamifop, Metamitron, Metazachlor, Methabenzthiazuron, Methiozolin, Methyldymron, Metobromuron, Metolachlor, Metosulam, Metoxuron, Metribuzin, Metsulfuron-methyl, Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonic acid, Pendimethalin, Pendralin, Penoxsulam, Pentoxazone, Pethoxamid, Phenmedipham, Picloram, Picolinafen, Pinoxaden, Piperophos, Pretilachlor, Primisulfuron-methyl, Profluazol, Profoxydim, Prometryn, Propachlor, Propanil, Propaquizafop, Propisochlor, Propoxycarbazone-sodium, Propyrisulfuron, Propyzamide, Prosulfocarb, Prosulfuron, Pyraclonil, Pyraflufen-ethyl, Pyrazolate, Pyrazosulfuron-ethyl, Pyrazoxyfen, Pyribenzoxim, Pyributicarb, Pyridafol, Pyridate, Pyriftalid, Pyriminobac-methyl, Pyrithiobac-sodium, Quinclorac, Quinmerac, Quinoclamine, Quizalofop-ethyl, Quizalofop-P-ethyl, Quizalofop-P-tefuryl, Rimsulfuron, Sethoxydim, Simazine, Simetryn, S-Metolachlor, Sulcotrione, Sulfentrazone, Sulfometuron-methyl, Sulfosate, Sulfosulfuron, Tebuthiuron, Tepraloxydim, Terbuthylazine, Terbutryn, Thenylchlor, Thiazopyr, Thifensulfuron-methyl, Thiobencarb, Tiocarbazil, Tralkoxydim, Triallate, Triasulfuron, Tribenuron-methyl, Triclopyr, Tridiphane, Trifloxysulfuron, Trifluralin, Triflusulfuron-Methyl-und Tritosulfuron.

### Weitere mögliche Mischungspartner sind

Pyroxasulfone, Pyroxsulam, Orthosulfamuron, Pyrimisulfan, Prohexadione-Calcium, Bencarbazone, SYN-523, IDH-100, SYP-249, Monosulfuron, Ipfencarbazone (HOK-201), Pyribambenz-Isopropyl, Tefuryltrione, Bencarbazone, Tembotrione, Pyrasulfotole und Thiencarbazone-Methyl.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

### Biologische Beispiele:

Herbizidwirkung und Safenerwirkung im Nachauflauf Samen bzw. Rhizomstücke von monound dikotylen Schadpflanzen und von Kulturpflanzen werden in Torftöpfen in sandiger Lehmerde ausgelegt, mit Erde abgedeckt und im Gewächshaus unter guten Wachstumsbedingungen angezogen. Alternativ hierzu werden im Paddy-Reisanbau vorkommende Schadpflanzen in Töpfen kultiviert, in denen Wasser bis zu 2 cm über der Bodenoberfläche steht. Zehn bis zwanzig Tage nach der Aussaat werden die Versuchspflanzen im ein bis Dreiblattstadium behandelt. Die als wasserlösliche Pulver oder Suspensionen formulierten erfindungsgemässen Herbizid-Safener-Zusammensetzungen sowie in parallelen Versuchen die entsprechend formulierten Einzelwirkstoffe werden in verschiedenen Dosierungen mit einer Wasseraufwandmenge von umgerechnet 300 I/ha auf die grünen Pflanzenteile gesprüht und nach 2-3 Wochen Standzeit der Versuchspflanzen im Gewächshaus unter optimalen Wachstumsbedingungen die Wirkung der Präparate optisch im Vergleich zu unbehandelten Kontrollen bonitiert. Bei Reis oder bei Schadpflanzen, die im Reisanbau vorkommen, werden die Wirkstoffe auch direkt ins Bewässerungswasser gegeben (Applikation in Analogie zur sogenannten Granulatanwendung) oder auf Pflanzen und ins Bewässerungswasser gesprüht. Die Versuche zeigen, dass durch den Einsatz der erfindungs-gemäßen Herbizid-Safener-Zusammensetzungen die Schäden an Kulturpflanzen wie Mais, Reis, Weizen oder Gerste im Vergleich zur Anwendung der einzelnen Herbizide ohne Safener wesentlich - d.h. um 30% bis zu 100% - reduziert werden. Gleichzeitig wird die Wirkung des Herbizids an wirtschaftlich bedeutenden Schad-pflanzen nicht oder nicht wesentlich beeinträchtigt, so dass eine gute herbizide Nachauflaufwirkung gegen ein breites Spektrum von Ungräsern und Unkräutern erreicht werden kann. Beispielsweise konnte in Gerste für die Herbizid-Safener-Zusammensetzungen (A1-2)+(S2-1), (A1-7)+(S2-1), (A1-60)+(S1-1), (A1-60)+(S2-1), (A1-61)+(S1-1), (A1-61)+(S2-1), (A2-4)+(S1-1), (A4-7)+(S1-1), (A4-7)+(S2-1) eine gute Verträglichkeit gegenüber den Kulturpflanzen bei gleichzeitig guter herbizider Nachauflaufwirkung gegen ein breites Spektrum von Schadpflanzen erzielt werden.

In Weizen konnte beispielsweise für die Herbizid-Safener-Zusammensetzungen (A1-2)+(S1-1), (A1-2)+(S1-11), (A1-7)+(S1-1), (A1-7)+(S2-1), (A1-7)+(S1-11), (A1-60)+(S1-1), (A1-60)+(S2-1), (A1-61)+(S1-1), (A1-61)+(S1-11), (A1-61)+(S2-1), (A2-4)+(S2-1), (A4-7)+(S1-1), (A4-7)+(S2-1) eine gute Verträglichkeit gegenüber den Kultur-pflanzen bei gleichzeitig guter herbizider Nachauflaufwirkung gegen ein breites Spektrum von Schadpflanzen erzielt werden. In Mais konnte beispielsweise für die Herbizid-Safener-Zusammensetzungen (A1-7)+(S1-11), (A1-7)+(S4-1), (A1-8)+(S1-11), (A1-8)+(S4-1), (A1-24)+(S1-11), (A1-24)+(S4-1), (A1-61)+(S1-11) eine gute Verträglichkeit gegenüber den Kulturpflanzen bei gleichzeitig guter herbizider Nachauflaufwirkung gegen ein breites Spektrum von Schadpflanzen erzielt werden

### Herbizidwirkung und Safenerwirkung im Vorauflauf

Samen bzw. Rhizomstücke von monound dikotylen Unkrautpflanzen und Kulturpflanzen wurden in Torftöpfen in sandiger Lehmerde ausgelegt und mit Erde abgedeckt. Die als wasserlösliche Pulver oder Suspensionen formulierten erfindungsgemässen Herbizid-Safener-Zusammensetzungen sowie in parallelen Versuchen die entsprechend formulierten Einzelwirkstoffe wurden dann in verschiedenen Dosierungen mit einer Wasseraufwandmenge von umgerechnet 600 bis 800 I/ha auf die Oberfläche der Abdeckerde appliziert. Nach der Behandlung wurden die Töpfe im Gewächshaus aufgestellt und unter guten Wachstumsbedingungen für die Unkräuter und die Kulturpflanzen gehalten. Die optische Bonitur der Pflanzenbzw. Auflaufschäden erfolgte nach dem Auflaufen der Versuchspflanzen nach einer Versuchszeit von 2 bis 4 Wochen im Vergleich zu unbehandelten Kontrollen. In Mais konnte beispielsweise für die Herbizid-Safener-Zusammen-setzungen (A1-7)+(S1-11), (A1-7)+(S4-1), (A1-8)+(S1-11), (A1-8)+(S4-1), (A1-24)+(S1-11), (A1-24)+(S4-1), (A1-61)+(S1-11), (A1-61)+(S4-1) eine gute Verträglichkeit gegenüber den Kulturpflanzen bei gleichzeitig guter herbizider Vorauflaufwirkung gegen ein breites Spektrum von Schadpflanzen erzielt werden.

### Saatgutbehandlung

Saatkörner von Kulturpflanzen wurden mit den als Suspensions- oder Emulsionskonzentraten formulierten Safenern und Wasser in Flaschen gemischt und gut geschüttelt, so dass die Saatkörner gleichmässig mit der Formulierung des jeweiligen Safeners beschichtet wurden. Die Saatkörner bzw. die aufgelaufenen Pflanzen wurden dann im Vorauflauf oder Nachauflaufverfahren mit Herbiziden behandelt. Dabei zeigten zahlreiche erfindungsgemäße Herbizid-Safener-Zusammensetzungen eine gute Verträglichkeit gegenüber den Kulturpflanzen bei gleichzeitig guter herbizider Wirkung gegen ein breites Spektrum von Schadpflanzen.

## Patentansprüche

1. Herbizid-Safener-Zusammensetzungen, enthaltend
(A) eine oder mehrere Verbindungen der Formel (1) oder deren Salze, 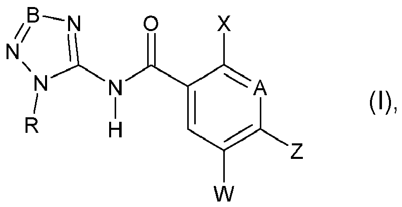 worin die Symbole und Indizes folgende Bedeutungen haben:
A bedeutet N oder CY,
B bedeutet N oder CH,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₃-C₆)-alkyl, Halogen-(C₃-C₆)-cycloalkyl-(C₃-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocylyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-AlkylS(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt, oder
Z kann auch Wasserstoff, (C₁-C₆)-Alkyl oder (C₁-C₆)-Alkoxy bedeuten, falls Y für den Rest S(O)ₙR² steht,
W bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₇)-Cycloalkyl, (C₃-C₇)-Halogencycloalkyl, (C₁-C₆)-Alkoxy, (C₁-C₆)-Halogenalkoxy, S(O)ₙ-(C₁-C₆)-Alkyl, S(O)ₙ-(C₁-C₆)-Halogenalkyl, (C₁-C₆)-Alkoxy-(C₁-C₄)-alkyl, (C₁-C₆)-Alkoxy-(C₁-C₄)-halogenalkyl, Halogen, Nitro, NR³COR³ oder Cyano,
R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Hydroxy, Nitro, Cyano, SiR⁵₃, PO(OR⁵)₂, S(O)ₙ-((C₁-C₆)-Alkyl, S(O)ₙ-(C₁-C₆)-Halogenalkyl, (C₁-C₆)-Alkoxy, Halogen-(C₁-C₆)-alkoxy, N(R³)₂, COR³, COOR³, OCOR³, NR³COR³, NR³SO₂R⁴, O(C₁-C₂)-Alkyl-(C₃-C₆)-Cycloalkyl, (C₃-C₆)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder
R bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl, wobei Heterocyclyl n Oxogruppen trägt,
Q bedeutet O, S, oder NR³,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl wobei die 21 letztgenannten Reste jeweils durch s Reste aus der Gruppe bestehend aus Cyano, Halogen, Nitro, Rhodano, OR³, S(O)ₙR⁴, N(R³)₂, NR³OR³, COR³, OCO_{R}³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 21 letztgenannten Reste jeweils durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet (C₁-C₄)-Alkyl,
n bedeutet 0, 1 oder 2, und
s bedeutet 0, 1, 2 oder 3,
sowie
(B) einen oder mehrere Safener.

2. Herbizid-Safener-Zusammensetzungen nach Anspruch 1, worin A bedeutet N oder CY,
B bedeutet N oder CH,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, OR¹, COOR¹, OSO₂R², S(O)ₙR², SO₂OR¹, SO₂ N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder
Z kann auch Wasserstoff, (C₁-C₆)-Alkyl oder (C₁-C₆)-Alkoxy bedeuten, falls Y für den Rest S(O)ₙR² steht,
W bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Nitro, Cyano, SiR⁵₃, P(OR⁵)₃, S(O)ₙ-(C₁-C₆)-Alkyl, (C₁-C₆)-Alkoxy, Halogen-(C₁-C₆)-alkoxy, N(R³)₂, COR³, COOR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder
R bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
Q bedeutet O, S, oder NR³,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die sechzehn letztgenannten Reste jeweils durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese sechzehn letztgenannten Reste jeweils durch s Reste aus der Gruppe bestehend aus Cyano, Halogen, Nitro, OR³, S(O)ₙR⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl,
R⁵ bedeutet Methyl oder Ethyl,
n bedeutet 0, 1 oder 2, und
s bedeutet 0, 1, 2 oder 3.

3. Herbizid-Safener-Zusammensetzungen nach Anspruch 1 oder 2, worin A bedeutet N oder CY,
B bedeutet N oder CH,
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Halogen-(C₁-C₆)-alkyl, (C₃-C₆)-Cycloalkyl, S(O)ₙR², 1,2,4-Triazol-1-yl, oder
Z kann auch Wasserstoff, Methyl, Methoxy oder Ethoxy bedeuten, falls Y für den Rest S(O)ₙR² steht,
W bedeutet Wasserstoff, Methyl, Ethyl, Methoxymethyl, Methoxy, Fluor, Chlor oder S(O)ₙCH₃,
R bedeutet (C₁-C₈)-Alkyl, Halogen-(C₁-C₈)-alkyl, (C₂-C₈)-Alkenyl, Halogen-(C₂-C₈)-alkenyl, (C₂-C₈)-Alkinyl, Halogen-(C₂-C₈)-alkinyl, wobei diese sechs vorstehend genannten Reste jeweils durch s Reste aus der Gruppe bestehend aus Cyano, S(O)ₙ-(C₁-C₆)-Alkyl, (C₁-C₆)-Alkoxy, Halogen-(C₁-C₆)-alkoxy, COR³, COOR³, OCOR³, NR³COR³, NR³SO₂R⁴, (C₃-C₆)-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt, oder
R bedeutet durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Phenyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die sechzehn letztgenannten Reste jeweils durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet jeweils durch s Reste aus der Gruppe bestehend aus Halogen und
OR³ substituiertes (C₁-C₆)-Alkyl, (C₃-C₆)-Cycloalkyl oder (C₃-C₆)-Cycloalkyl-(C₁-C₆)-alkyl,
R³ bedeutet Wasserstoff oder (C₁-C₆)-Alkyl,
R⁴ bedeutet (C₁-C₆)-Alkyl,
R⁵ bedeutet Methyl oder Ethyl,
n bedeutet 0, 1 oder 2, und
s bedeutet 0, 1, 2 oder 3.

4. Herbizid-Safener-Zusammensetzungen nach einem der Ansprüche 1 bis 3, worin
A bedeutet CY,
B bedeutet N,
X bedeutet Chlor, Methyl, Ethyl, Propyl, cyclo-Propyl, Methoxy oder SO₂CH₃,
Y bedeutet Wasserstoff, CH₂OCH₃, CH₂OCH₂CF₃, CH₂OC₂H₄OCH₃, 4,5-Dihydro-1,2-oxazol-3-yl, 5-Methoxymethy-4,5-dihydro-1,2-oxazol-3-yl, Pyrazol-1-yl, OMe, OEt, OPr, OiBu, OCH₂cPr, OC₂H₄OCH₃, SO₂ CH₃, S(O)CH₃ oder SCH₃, SO₂Et, S(O)Et oder SEt,
Z bedeutet Trifluormethyl, SO₂CH₃, SO₂Et, Chlor oder Brom,
W bedeutet Wasserstoff, und
R bedeutet Methyl, Ethyl, Propyl oder Methoxyethyl.

5. Herbizid-Safener-Zusammensetzungen nach einem der Ansprüche 1 bis 4, worin
A bedeutet CY,
B bedeutet N
X bedeutet Chlor,
Y SO₂CH₃, SOCH₃ oder SO₂Et,
Z bedeutet Wasserstoff oder Methyl,
W bedeutet Wasserstoff, und
R bedeutet Methyl.

6. Herbizid-Safener-Zusammensetzungen nach einem der Ansprüche 1 bis 5, worin
A bedeutet CY,
B bedeutet N
X bedeutet Chlor oder Brom,
Y bedeutet Wasserstoff, Methyl, SO₂CH₃ oder SCH₃, Z bedeutet Wasserstoff, SO₂CH₃ oder SCH₃,
W bedeutet Methyl, und
R bedeutet Methyl oder Ethyl.

7. Herbizid-Safener-Zusammensetzungen nach einem der Ansprüche 1 bis 3, worin
A bedeutet CY,
B bedeutet CH,
X bedeutet Chlor oder Methyl,
Y bedeutet 4,5-Dihydro-1,2-oxazol-3-yl, Pyrazol-1-yl, OC₂H₄OCH₃ oder SO₂CH₃, Z bedeutet Trifluormethyl, SO₂CH₃, SO₂Et oder Chlor,
W bedeutet Wasserstoff, und
R bedeutet Methyl.

8. Herbizid-Safener-Zusammensetzungen nach einem der Ansprüche 1 bis 3, worin
A bedeutet N,
B bedeutet N,
X bedeutet Chlor, Brom, SO₂CH₃, Methoxymethyl, OCH₂cPr, OC₂H₄OCH₃ oder Methyl,
Z bedeutet Trifluormethyl,
W bedeutet Wasserstoff, und
R bedeutet Methyl oder Ethyl.

9. Herbizid-Safener-Zusammensetzungen nach einem der Ansprüche 1 bis 8, enthaltend mindestens einen Safener ausgewählt aus der Gruppe bestehend aus Mefenpyr-diethyl, Fenchlorazol, Isoxadifen-ethyl, Cloquintocet-mexyl, Dichlormid, 3-Dichloracetyl-2,2,5-trimethyl-1,3-oxazolidin, Benoxacor, 3-Dichloracetyl-1-oxa-3-azaspiro[4,5]decan, 1-Dichloracetyl-azepan, Furilazol, ((R)-3-Dichloracetyl-5-(2-furyl)-2,2-dimethyloxazolidin), Cyprosulfamid, N-Isopropyl-4-sulfamoylbenzamid--1-(2-methoxyphenyl)ethanon, 1-[4-(N-2-Methoxybenzoylsulfamoyl)phenyl]-3-methylharnstoff, Oxabetrinil, Fluxofenim, Cyometrinil, 1,8-Naphthalindicarbonsäureanhydrid, Fenclorim, Flurazole, Dietholate, Dimepiperate, Daimuron und Cumyluron.

10. Herbizid-Safener-Zusammensetzungen nach einem der Ansprüche 1 bis 8, enthaltend mindestens einen Safener ausgewählt aus der Gruppe bestehend aus Daimuron, Benoxacor, Furilazol, Fluxofenim, Fenchlorazol(-ethylester), Mefenpyr-diethyl, Cloquintocet-mexyl, Isoxadifen-ethyl, Cyprosulfamide, Flurazole, Oxabetrinil, Dichlormid und Dietholate.

11. Herbizid-Safener-Zusammensetzungen nach einem der Ansprüche 1 bis 8, enthaltend mindestens einen Safener ausgewählt aus der Gruppe bestehend aus: Mefenpyr-diethyl, Isoxadifen-ethyl, Cyprosulfamide, Fenchlorazol-ethylester, Benoxacor, Cloquintocet-mexyl, Fluxofenim und Furilazol.

12. Verfahren zur Bekämpfung von Schadpflanzen in Kulturen von Nutzpflanzen, **dadurch gekennzeichnet, dass** man eine herbizid wirksame Menge einer Zusammensetzung gemäß einem oder mehreren der Ansprüche 1 bis 11 auf die Schadpflanzen, Pflanzen, Pflanzensamen oder die Fläche, auf der die Pflanzen wachsen, aufbringt.

13. Verfahren gemäß Anspruch 12, **dadurch gekennzeichnet, dass** die Nutzpflanzen aus der Gruppe Zuckerrohr, Mais, Weizen, Roggen, Gerste, Hafer, Reis, Sorghum, Baumwolle und Soja stammen.

14. Verfahren gemäß Anspruch 12 oder 13, **dadurch gekennzeichnet, dass** die Nutzpflanzen gentechnisch verändert sind.
